(19) Europäisches Patentamt
European Patent Office
Office européen des brevets

(11) Publication number: 0 010 807
B1

(12) EUROPEAN PATENT SPECIFICATION

(45) Date of publication of patent specification: 26.01.83

(21) Application number: 79200612.4

(22) Date of filing: 25.10.79

(51) Int. Cl.[3]: C 07 C 53/128, C 07 C 51/00, C 07 C 69/22, C 07 C 103/44, C 07 D 295/12, C 08 G 65/32, C 08 K 5/04, C 08 K 5/16, C 08 L 91/00 //C10M3/00, B01J23/34, B29C1/04

# (54) Derivatives of branched-chain monocarboxylic acids, engine lubricating oils, lubricant compositions for polymers, wax compositions in which these derivatives are incorporated and a process for preparing said branched-chain monocarboxylic acids.

(30) Priority: 26.10.78 NL 7810668
11.07.79 NL 7905409

(43) Date of publication of application:
14.05.80 Bulletin 80/10

(45) Publication of the grant of the patent:
26.01.83 Bulletin 83/4

(84) Designated Contracting States:
BE CH DE FR GB IT NL SE

(56) References cited:
FR - A - 2 194 680
US - A - 3 864 368
US - A - 4 012 357

(73) Proprietor: Akzo N.V.
Velperweg 76
NL-6824 BM Arnhem (NL)

(72) Inventor: Zeilstra, Jacobus Johannes
Doetinchemseweg 4
NL-7048 AA Wijnbergen (NL)
Inventor: Bik, Joannes Dominicus
Sophia van Württemberglaan 3
NL-6961 EV Eerbeek (NL)
Inventor: De Klein, Willem Jan
Hoflaan 12
NL-6953 AM Dieren (NL)
Inventor: Vis, Jan
Vondellaan 116
NL-6901 ML Zevenaar (NL)

(74) Representative: Sieders, René et al,
P.O. Box 314
NL-6800 AH Arnhem (NL)

EP 0 010 807 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Derivatives of a mixture of monocarboxylic acids, at least part of which are branched-chain acids, engine lubricating oils, lubricant compositions for polymers and wax compositions in which these derivatives are incorporated

The invention relates to derivatives of a mixture of monocarboxylic acids, at least part of which are branched-chain acids, selected from the group consisting of

a) esters of phenols or aromatic, aliphatic or cycloaliphatic alcohols having at least 1 to 10 primary or secondary hydroxyl groups;

b) amides of ammonia or of aliphatic, cycloaliphatic or aromatic amines having at least 1 to 15 primary or secondary amino groups;

c) salts of alkali metals or alkaline earth metals, amphoteric metals, heavy metals or ammonium or of a compound containing a tertiary amino group; and to engine lubricating oils whose base fluid entirely or partly consists of one or more of said derivatives, and to lubricant compositions for polymers entirely or partly made up of one or more of these derivatives, and to wax compositions in which one or more of these derivatives are incorporated.

Derivatives of a mixture of monocarboxylic acids, at least part of which are branched-chain acids, are known to be used for various purposes. Ester mixtures of branched-chain and straight-chain monocarboxylic acids are described in, inter alia, British Patent Specification 1 441 918. They are esters derived from a branched-chain polyol such as pentaerythritol or trimethylol propane and a mixture of 5 to 90 mole% of branched-chain monocarboxylic acids having 13 to 22 carbon atoms and 95 to 10 mole% of straight-chain or branched-chain monocarboxylic acids having 3 to 12 carbon atoms. These esters are said to be excellently suitable to be used in engine lubricating oils. Applicant has found, however, that for the last-mentioned use the known esters can be improved upon in several respects. This particularly applies to the pour point and the viscosity index. The present invention provides a new class of esters having a surprisingly low pour point and an extraordinarily good viscosity behaviour.

Amide derivatives of carboxylic acids and alkylene polyamines or hydroxyalkyl-substituted polyamines are described inter alia in United States Patent Specification 3 163 603.

The carboxylic acid described in it is a dicarboxylic acid, viz. a hydrocarbon-substituted succinic acid. The hydrocarbon group contains at least 50 carbon atoms.

Although these products are satisfactorily dispersing agents, corrosion inhibitors and/or acid binders, there is yet a great need for products having similar properties, but improved solubility and improved rheological behaviour in mineral oils. The use of salts of the above-mentioned type and of mixtures of salts and esters of, in particular, higher fatty acids in lubricant compositions for polymers is described in, inter alia, the United States Patent Specification 4 029 682.

Applicant has found that lubricant compositions of a remarkably better quality are obtained if they are prepared from a mixture of acids having a product composition which totally differs from the one described in said patent specification.

Esters of branched-chain and straight-chain high molecular weight aliphatic monocarboxylic acids together with unesterified acids and/or salts (soaps) thereof often form part of synthetic wax compositions. It has now been found that incorporation into wax compositions of derivatives according to the present invention leads to waxes which display greatly improved properties for a very large number of uses thereof. The present invention provides a novel class of derivatives of branched-chain monocarboxylic acids having surprisingly good properties for the above mentioned uses.

The present invention consists in that the monocarboxylic acids are derived from the reaction product of an $\alpha$-olefin having 6 to 45 carbon atoms and acetic anhydride, and at least 10% by weight of the branched-chain acids conforms to the formula:

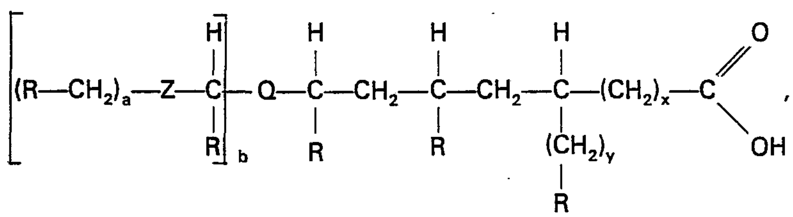
, wherein

y=0 or 2 and
x=0, if y=2
or x=2, if y=0
$R=CH_3(CH_2)_n$, where n represents an integer of from 3 to 42;
b=0 or 1, where,
if b=0, Q represents a hydrogen atom, and
if b=1, Q represents a $CH_2$-group, and
a=0 or 1, where

if a=0, Z represents a hydrogen atom, and
if a=1, Z represents a $CH_2$-group.

The process of reacting an $\alpha$-olefin having 6 to 45 carbon atoms with acetic anhydride is preferably carried out at a temperature in the range of 100° to 140°C in the presence of a catalytic amount of an at least trivalent manganese compound.

It should be added that a process of the above type is disclosed in French Patent Specification 2 194 680. It relates to a process for the preparation of straight-chain carboxylic acids by reacting an (ar)-alkene in the presence of a manganese compound, which is at least trivalent, with a carbonyl compound having at least one hydrogen atom attached to the alpha-carbon atom. Telomerization is characterized as an undesired side reaction (page 4, lines 10—28).

The $\alpha$-olefin may consist of a pure olefin fraction, such as 1-octene, or of a mixture of $\alpha$-olefins having 6 to 45 carbon atoms. If use is made of a mixture of $\alpha$-olefins, then the number for n in each separate R-radical may, independently of the other R-radicals in the first-mentioned structural formula of the acid, assume any value equal to the number of carbon atoms minus two of an $\alpha$-olefin present in the mixture. The most favourable results are generally obtained at a reaction temperature in the range of 115° to 125°C in the presence of manganese acetate as initiator.

To prevent oxidation of the substrate the concentration of the manganic acetate is preferably chosen between $10^{-3}$ and $10^{-10}$ moles per liter. The concentration of the olefin fraction is dependent on the desired percentage of branched-chain monocarboxylic acids in the reaction product, which percentage is partly determined by the olefin starting fraction. If use is made of an olefin starting fraction having not more than 20 carbon atoms, it is preferred — in view of the above-described field of application — that use should be made of a relatively high percentage of branched-chain acids. If, however, use is made of an olefin fraction having 20 to 45 carbon atoms, then more preference is found to be given to a mixture of monocarboxylic acids containing at least 70% by weight of the addition product of 1 mole of olefin to 1 mole of acetic acid. In all cases the reaction conditions will be so chosen that at least 10% by weight of the branched-chain acids conforms to the above-mentioned structural formula.

It should be added that also in the United States Patent Specifications 3 988 330, 4 012 357 and 4 029 682 mention is made of an addition reaction of $\alpha$-olefins with a monocarboxylic acid under the influence of a radical initiator. The olefins always contain 22 or more carbon atoms. The reaction is carried out in the presence of a propionic acid or a higher carboxylic acid as solvent and inorganic or organic peroxides as radical initiator. Upon termination of the reaction the addition product is esterified with mono- and/or polyfunctional alcohols. A disadvantage to this process consists in that the conversion of the addition reaction is not higher than about 50 to 70%, which results in a higher percentage of olefin fraction in the end product which cannot be separated from it on a commercial scale. The resulting telomeric monocarboxylic acids not only strongly differ from the derivatives according to the invention as far as structure is concerned (exclusively $\alpha$-branching) but also their product distribution is entirely different.

For the preparation of branched-chain monocarboxylic acids according to the first-mentioned formula, use is made of a molar ratio of converted olefin to manganic acetate of at least 4. It has been found that under these last-mentioned conditions the % by weight composition of the mixture of telomeric acids is for $n \leqslant 17$ only dependent on the molar ratio of $\alpha$-olefin to manganic acetate and the concentration of $\alpha$-olefin during the reaction. With a monocarboxylic acid obtained by reacting one $\alpha$-olefin with acetic acid being indicated by $R_1$, a monocarboxylic acid obtained by reacting two $\alpha$-olefins by $R_2$, a monocarboxylic acid obtained by reacting three $\alpha$-olefins by $R_3$, etc., it is found that respectively before and after removal of the $R_1$-fraction the following is a typical example of a weight distribution that may be obtained.

| | before distillation weight % | after distillation weight % |
|---|---|---|
| $R_1$ | 30,7 | 0,3 |
| $R_2$ | 20,4 | 19,8 |
| $R_3$ | 21,4 | 33,6 |
| $R_4$ | 13,0 | 21,5 |
| $R_5$ | 9,4 | 15,9 |
| $R_6$ | 5,1 | 8,8 |

The structural formulae of $R_3$, $R_4$ and $R_5$ all correspond to the first-mentioned formula. $R_1$ is an unbranched acid of the formula $R\ (CH_2)_3\ COOH$ and for the above-mentioned reasons it is preferably removed from the reaction mixture in the case of esterification with an aliphatic alcohol having only one primary hydroxyl group. It will be obvious that in the case of esterification with a polyol $R_1$ also can partly be removed from the reaction mixture for instance by distillation. The $R_2$ fraction is formed by two acids of the formula:

$(R—CH_2CH_2)_2C^HCOOH$ or 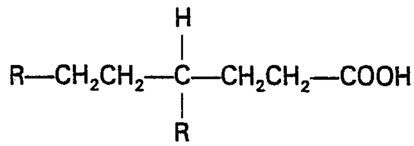

To a man skilled in the art it is obvious that especially when use is made of an olefin starting fraction having 30 or more carbon atoms, it is not possible in actual practice to separate the linear acids from the acids having a very high molecular weight and a high degree of telomerization. It has been found that the use of mixtures of derivatives of these branched-chain and linear carboxylic acids in polymers, wax compositions, etc., employed for all the above-mentioned purposes lead to compositions having unexpectedly favourable properties, which remarkably favourably compare with the known compositions that only contain derivatives of straight-chain fatty acids.

The following is a typical example of a weight distribution of monocarboxylic acids obtained from a $C_{30+}$ olefin mixture.

| degree of telomerization (m) | weight % |
|---|---|
| m=1 | 78,0 |
| m=2 | 6,3 |
| m=3 | 6,5 |
| m=4 | 4,0 |
| m=5 | 3,1 |
| m⩾6 | 2,0 |

The commercially available olefin fractions having 20 to 45 carbon atoms are found to contain 60 to 80% by weight of $\alpha$-olefins and for the rest predominantly consist of vinylidene compounds. The resulting carboxylic acids are $\gamma$-branched monocarboxylic acids, with the acid having the formula

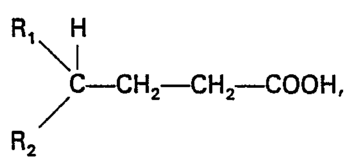

where $R_1$ and $R_2$ represent linear alkyl groups which together have the same number of carbon atoms as the group R.

Separation of these vinylidene groups-containing fractions from the $\alpha$-olefins would give rise to so many technological problems that it must be considered impracticable for economic reasons. It has been found, however, that for most uses products having exceptionally good properties are obtained if besides the derivatives prepared from the branched-chain acids of the above formulae 40 to 60 per cent by weight of the derivatives is prepared from linear aliphatic monocarboxylic acids, with the acid having the formula $RCH_2CH_2CH_2COOH$, where R represents a $CH_3(CH_2)_n$ group with n representing an integer from 17 to 42.

The proportion of derivatives of $\gamma$-branched carboxylic acids, with the acid having the formula:

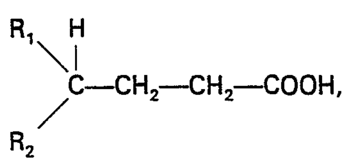

where $R_1$ and $R_2$ represent linear alkyl groups which together have the same number of carbon atoms as the group R, will vary between 30 and 40 per cent by weight.

It has been found that for the preparation of lubricant compositions for engine oils, and internal and external lubricants for polymers and lubricants used in the manufacture of textile materials use may with advantage be made of derivatives of branched-chain acids according to the invention, where n represents an integer from 3 to 17.

For the preparation of components in synthetic wax compositions it is preferred to make use of derivatives of branched-chain acids of the first-mentioned formula where n represents an integer in the range of 17 to 42.

When the lubricant compositions are to be used for engine oils, it is preferred that use should be made of aliphatic esters of branched-chain acids of the first-mentioned formula, where n represents an integer of from 3 to 11. As additional advantage thereof may be mentioned that they have a relatively high average molecular weight, as a result of which the loss of weight of a lubricating oil having in the base fluid the esters according to the invention compares extremely favourably with the known lubricating oils derived from synthetic esters.

In view of the high thermal resistance, the high viscosity index and resistance to oxidation of the esters according to the invention there will be need for fewer or less costly additives, which may greatly influence the final price of the finished product.

For use in engine lubricating oils it is preferred according to the invention to employ esters derived from an aliphatic alcohol having 1 to 18 carbon atoms. This may be a monofunctional alcohol such as n-butanol or a polyfunctional alcohol. The former has the advantage that the esterification proceeds at a higher speed and without further great difficulties. In the case of polyfunctional alcohols, however, higher molecular and even less volatile products may be obtained. Particularly attractive esters are found to be produced if the aliphatic alcohol is a branched-chain polyalcohol having 2 to 4 primary hydroxyl groups. In this connection mention is made of the favourable properties of esters of which the aliphatic polyalcohol is neopentyl glycol. Other esters having particularly good properties are obtained by using trimethylol ethane or dipentaerythritol.

In view of their high thermal stability, preference is given to esters derived from such aliphatic polyalcohols as trimethylol propane or pentaerythritol.

For the preparation of diesters of acids having the first-mentioned formula it is of advantage to make use of the epoxides instead of the corresponding alcohols. Particularly if instead of the telomeric acids the corresponding anhydrides are used, the reaction in the presence of 1 mole per cent of a tetraalkyl ammonium bromide such as tetrabutyl ammonium bromide proceeds very rapidly. It will be evident that in the esterification use may be made of pure telomeric acids or of a mixture thereof with other acids, such as acetic acid, lauric acid or oleic acid. As examples of suitable epoxides for the preparation of diesters may be mentioned ethylene oxide, propylene oxide, the epoxides derived from $\alpha$-olefins having 3 to 45 carbon atoms, the epoxides derived from other unsaturated alkenes, esters of 2,3-epoxy-1-propanol, epoxidized natural oils, such as epoxidized soy bean oil, cyclic epoxides and diepoxides. In addition to being suitable for the preparation of diesters, the epoxides may be used for the preparation of esters having a high hydroxyl number.

It has been found that many of the derivatives according to the present invention with n representing an integer from 3 to 17 provide liquid lubricant formulations. Especially the highly branched derivatives show an external lubricating effect and an unexpected degree of clarity in PVC compositions.

Suitable phenols that may be used for the preparation of the ester derivatives according to the invention include phenol, cresol, xylenol, mesitol, durenol, thymol, naphthol, resorcinol, hydroquinone, bisphenols such as 4,4'-oxydiphenol, 4,4'-isopropylidene diphenol, 4,4'-methylene diphenol and biphenyl-4,4'-diol. They may optionally be substituted with, for instance, alkyl or alkoxy groups or halogen. Suitable aromatic hydroxyl compounds include benzyl alcohol, tolyl alcohol (=methylphenyl carbinol), phenethyl alcohol, salicyl alcohol, 2-naphthalene ethanol, phenylpropyl alcohol and cinnamyl alcohol.

Suitable aliphatic or cycloaliphatic alcohols include monohydric alcohols, di- en higher polyhydric alcohols and ether alcohols, which may be mono- or polyfunctional. Optionally, they may contain ethylenically unsaturated groups or other substituents, such as alkyl, alkoxy, halogen or heterocyclic groups such as in furfuryl alcohol. They may contain 1 to 60 or more carbon atoms. Suitable ester derivatives are obtained by making use of methanol, ethanol, n-propanol, isopropanol, n-butanol, t-butanol, iso-amylalcohol, n-hexanol, cyclohexanol, 2-cyclohexene-1-ol, 2-ethylhexanol, n-octanol, isodecanol, capryl alcohol, lauryl alcohol, myristyl alcohol, cetyl alcohol, stearyl alcohol and oxo alcohols such as tridecyl alcohol, which substantially consists of tetramethyl-1-nonanol and hexadecyl alcohol which is made up of a composite mixture of primary alcohols and may be characterized as 2,2-dialkyl ethanols in which the alkyl groups substantially consist of methyl-branched $C_6$ and $C_8$ radicals. As examples of aliphatic polyols suitable to be used for the preparation of the ester derivatives may be mentioned: ethylene glycol, 1,2-propylene glycol, 1,3-propylene glycol, 1,2-butanediol, 1,4-butanediol,, 1,5-pentanediol, 3-methyl-1,5-pentane diol, 3-methyl-1,5-pentane diol, 2,3-dimethyl-2,3-butanediol, trimethylol propane, mannitol, sorbitol, glycerol and pentaerythritol. The preparation of the novel ester

derivatives according to the invention also may be carried out with the use of ether alcohols.

The ether alcohols may be monofunctional or polyfunctional and contain 2 to 8 condensed polyol units. Suitable ether alcohols include diethylene glycol, triethylene glycol, tetraethylene glycol, diethylene glycol monomethyl ether, diethylene glycol monoethyl ether, triethylene glycol monomethyl ether, butoxyethanol, butylene glycol monobutyl ether, dipentaerythritol, tripentaerythritol, tetrapentaerythritol, diglycerol, tetraglycerol, pentaglycerol, hexaglycerol, heptaglycerol and octaglycerol. Also suitable compounds have been found to be mono- or diesters of the polyalkylene oxide glycols having 2 to 50 alkylene oxide units each containing 2 to 4 carbon atoms. Examples thereof include polyethylene oxide glycol having a molecular weight of 500 to 2000 and polytetrahydrofuran having a molecular weight of 600 to 2000. For use as lubricant in polymers or resin compositions not all free hydroxyl groups need be esterified. Also partially esterified polyols are within the scope of the present invention. It is preferred that at least 50% of the available hydroxyl groups are converted into esters.

The esters of the present invention are also suitable to be used as dispersant in lubricating oils. Especially the equimolar reaction products of the acids according to the first-mentioned formula and pentaerythritol and/or trimethylol propane can very effectively be used for the above-mentioned purpose.

It has been found that resin compositions having remarkably good lubricating properties are obtained if use is made of esters of aliphatic polyols and ether polyols having 2 to 12 carbon atoms and 2 to 8 primary or secondary hydroxyl groups. Special mention should be made in this respect of esters derived from ethylene glycol, neopentyl glycol, pentaerythritol, dipentaerythritol, tripentaerythritol, glycerol, and/or di-, tri-, and/or tetraglycerol. The acid number of these esters is preferably lower than 30 and the hydroxyl number lower than 40.

It will be clear that also mixtures of esters derived from at least one acid of the first-mentioned formula and some other branched or non-branched acid may be suitable for use in polymer and/or engine lubricating compositions according to the invention. In the preparation of the acids of the first-mentioned formula also other branched and non-branched fatty acids are obtained. It has been found that the presence of esters derived from a polyol having at least 2 to 10 primary or secondary hydroxyl groups, at least one branched-chain acid having the first-mentioned formula and a branched acid having the formula

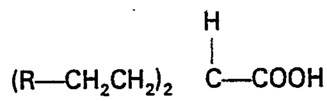 and/or 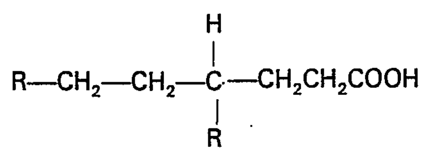

where R has the same meaning as in the first mentioned formula, leads to obtaining products having particularly good lubricating properties. Besides, it has been found that also esters derived from a polyol having at least 2 to 10 primary or secondary hydroxyl groups, at least one branched-chain acid of the first-mentioned formula and an unbranched aliphatic monocarboxylic acid having 7 to 47 carbon atoms are very suitable for use in polymer and/or engine lubricating compositions according to the invention. What has been said above about the esters of polyalcohols applies, mutatis mutandis, to all derivatives of the present invention prepared from a polyfunctional compound such as a polyamine or a polyisocyanate.

It will further be clear that not only the derivatives of the monocarboxylic acids of the first-mentioned formula and monofunctional alcohols, amines, isocyanates and the like, but also mixtures containing derivatives of other branched or non-branched monocarboxylic acids may be used for one of the above-mentioned purposes. For a man skilled in the art it will not be difficult, on the basis of the physical properties of the separate properties, to establish whether or not it is possible to use mixtures also containing derivatives of other branched or non-branched monocarboxylic acids. The preparation of the acids according to the present invention also leads to the formation of straight-chain aliphatic carboxylic acids. It has been found that for a large number of uses they need not be removed from the mixture. It should be added that the use in lubricating oils of esters derived from a monofunctional alcohol and an unbranched aliphatic monocarboxylic acid having 2 or more carbon atoms should as much as possible be avoided both because of their relatively high volatility and their tendency to increase the pour point.

The esters according to the invention are prepared by esterifying an alcohol or a mixture of alcohols in a manner known in itself, in which process per alcohol at least one branched-chain acid of the first-mentioned formula is present. As indicated above, also other branched or non-branched acids may be present. The esterification reaction may be carried out in the usual manner. The reaction mixture is heated to a temperature of 100° to 300°C in the presence or not of a catalyst, the water evolved in the reaction being discharged. The esterification is usually carried out at a temperature in the range of 140° to 280°C. Optionally, use may be made of an esterification catalyst. This may be an acid

such as sulphuric acid, phosphoric acid, alkylsulphonic acids and arylsulphonic acids such as p-toluene sulphonic acid and methane sulphonic acid, and a variety of metal compounds such as dibutyl tin oxide, tetrabutyl titanate, zinc acetate, stannooxalate, iron oxide, ferristearate, manganostearate, cobalt (II) stearate and manganoacetate.

The catalyst is usually employed in an amount of 0,1 to 1% by weight, based on the reaction mixture. Optionally, use may be made of an inert thinner which together with water forms an azeotrope, such as benzene, toluene or xylene.

In the process use is generally made of stoichiometric amounts of acid and alcohol, although in the esterification with the more volatile alcohols the latter also may be used in excess. By the end of the reaction the excess is removed from the reaction mixture by distillation. Esterification may take place at atmospheric pressure, but it is also possible to have it carried out at reduced pressure (2—50 mm Hg). Under such conditions the excess alcohol and water can readily be removed upon completion of the reaction. The resulting esters can as a rule directly be used for one of the above purposes. Under some circumstances, however, it may be advisable also to apply a purification step, for instance by treating the compositions with bleaching earth, ozone, peroxide, hyprochlorite or some other suitable bleaching agent. The preparation also may include a treatment with active carbon.

The esters derived from aliphatic monoalcohols having 1 to 8 carbon atoms and a monocarboxylic acid of the first-mentioned formula, where n=3 to 9, may be purified for instance by distillation.

For use in engine lubricating oils the final acid number should be lower than 0,2 and the hydroxyl number lower than 10.

Besides the above-mentioned esters also soaps (salts) and mixtures of esters and soaps (ester-soaps) prepared from an acid according to the first-mentioned formula are excellently suitable to be used in lubricating compositions of, more particularly, polyvinyl chloride and copolymers thereof and in lubricating oils. Useful soaps and ester-soaps prepared from the above-described higher molecular weight acids include those obtained using alkali metals, alkaline earth metals, amphoteric metals and heavy metals. Illustrative metals include: lithium, sodium, potassium, beryllium, magnesium, calcium, strontium, barium, copper, silver, zinc, cadmium, mercury, aluminium, iron, nickel, cobalt and the like. Especially useful metallic soaps for use in polymers are prepared from the metals of lithium, calcium, barium, magnesium, zinc, lead, cadmium or tin and mixtures thereof. For use in lubricating oils preference is given to soaps prepared from the acids according to the first mentioned formula, where n represents an integer of <20.

Especially preferred are the salts prepared from a metal selected from the group of calcium, barium and magnesium.

In lubricant compositions for engine oils or polymers, particularly polyvinyl chloride, use may be made of overbased formulations comprising one or more of the above salts.

Examples of processes according to which the overbased formulations (dispersions) may be prepared are described in U.S. Patents 2 585 520, 2 739 124, 2 889 279, 2 895 913, 3 149 074, 3 150 089, 3 235 494 and 3 629 109.

Metal contents of resins containing these products may range from low levels, about 0,1 weight% with certain ester-soaps, to as high as 15 wt.% or more for overbased formulations with the soaps of some of the heavier metals. The salts (soaps) are prepared in a known manner by the wet route of precipitation or by the dry route of melting. The preparation of the insoluble soaps derived from lower olefins may be effected by a double decomposition reaction in which first the acid is dissolved in hot water and subsequently neutralized with a sodium hydroxide solution in order to obtain the soluble sodium salt. Next, a solution of the respective heavy metal is slowly added with stirring. The insoluble metal salt instantaneously precipitates from the solution and is isolated therefrom by filtration.

This reaction is usually carried out at a temperature in the range of 50°C to 90°C and can only be applied in the preparation of soaps that give a watersoluble sodium salt. This is generally the case if n=3 to 7. In the preparation of the soaps use will generally be made of the direct procedure of heating the acid in the presence of a metal oxide, hydroxide or weakly acid salt.

Mixtures of esters and soaps are obtained by partial saponification of the acid, that is by reacting the carboxyl groups of the branched-chain acids with a metal compound and an aliphatic hydroxyl compound having 1 to 40, and preferably 1 to 12 carbon atoms and 1 to 10 primary or secondary hydroxyl groups. The reaction of the metal compound and the aliphatic hydroxyl compound with a mixture of the high molecular weight monocarboxylic acids may be carried out stepwise or in a single step. The monocarboxylic acids may first be contacted with the desired amount of metal compound in order partially to neutralize the acid and subsequently esterifying the remaining carboxyl groups by reaction with the hydroxyl compound.

Alternatively, first a part of the carboxyl groups may be esterified and the remaining part of the carboxyl groups be neutralized with the metal compound. It is preferred, however, to apply the single-step procedure. Esterification is effected under the same conditions as given above for the preparation of pure esters.

Not only salts of metals and tertiary amines such as triethylamine, pyridine and quinoline, but also amides of ammonia or of aliphatic, cycloaliphatic or aromatic amines and a branched-chain acid of the

first-mentioned formula are excellently suitable for use in lubricating oils or to replace the known amide waxes.

Suitable amines from which the amides are derived include: methylamine, ethylene diamine, butylamine, hexamethylene-1,6-diamine, p-phenylene diamine and 1,4-cyclohexyl diamine, methylethyl amine and 2-(N-methylamino) heptane. Also suitable for use are substituted amines, such as ethanol amine and butanol amine.

The amides of the present invention are not only suitable for use as dispersing agents, corrosion inhibitors and/or acid binders in lubricant compositions. It has been found that they lend themselves excellently to being employed as curing catalyst for epoxy resins. In addition, mention should be made of their use as surface active compound.

For use in lubricating oils preference is given to amide derivatives of acids of the first-mentioned formula, where n is an integer of from 3 to 23. Particularly it is considered that use should be made of amide derivatives of alkylene polyamines or hydroxyalkyl-substituted polyamines. Over the compounds described in the prior art such as U.S. Patent Specification 3 163 603 they have the advantage of improved solubility and improved rheological behaviour in mineral oils. They readily mix with mineral oil fractions which are usually employed as base fluid in lubricating compositions. It has been found that for most uses products having attractive properties are obtained, if the polyamine from which the amides are derived corresponds to the formula:

$$\underset{\substack{|\\A}}{H-N}\,(\text{alkylene}-\underset{\substack{|\\A}}{N})_n-H,$$

where n represents an integer of from 1 to 10, A is a hydrogen atom or a hydrocarbon group substituted or not with an amino group, and the alkylene group is a lower alkylene group having not more than 8 carbon atoms. Preference is especially given to ethylene diamine and the oligomers thereof.

As examples of other polyamines may be mentioned the following compounds or higher homologues thereof:

propylene diamine, decamethylene diamine, octamethylene diamine, di(heptamethylene) triamine, tripropylene tetramine, trimethylene diamine, di(trimethylene)triamine, 2-heptyl-3-(2 aminopropyl)imidazoline, 1,3-bis(2-aminoethyl)imidazoline, pyrimidine, 1-(2-aminopropyl)piperazine, 1,4-bis-(2-aminoethyl)piperazine and 2-methyl-1-(2-aminobutyl)piperazine.

Preference is generally given to amides derived from diethylene triamine, triethylene tetramine, tetraethylene pentamine and pentaethylene hexamine. The higher homologues may, of course, be composed of only one monomer, or of mixtures of two or more diamines.

Other amides having exceptionally favourable properties are obtained if the amides are derived from aminoethyl piperazine.

The hydroxyalkyl-substituted polyamines from which the amides according to the present invention may be derived are generally alkylene·polyamines having one or more hydroxyalkyl substituents on a nitrogen atom. The alkyl group of hydroxyalkyl-substituted polyamines generally has not more than 6 carbon atoms. Examples of such amines include N-(2-hydroxyethyl)-ethylene diamine, N,N'-bis(2-hydroxyethyl)ethylene diamine, 1-(2-hydroxyethyl)-piperazine, hydroxypropyl-substituted diethylene triamine, di-hydroxypropyl-substituted tetraethylene pentamine, and N-(3-hydroxypropyl)-tetramethylene diamine.

Other useful amide derivatives are the hydroxyalkyl-substituted alkylene amines obtained by condensation of the above-mentioned alkylene amines through amino groups or hydroxyl groups.

Condensation through the amino groups with $NH_3$ being split off results in a higher polyamine, and condensation through the hydroxyl groups results in products containing ether linkages.

The amide derivatives according to the present invention may be prepared from a pure carboxylic acid or a mixture of carboxylic acid according to the first-mentioned formula or from the homoanhydrides or mixed anhydrides thereof. At least about one equivalent of one of the above-mentioned amines is added, with the mixture being heated and the water formed being removed. The acylation reaction may be carried out at a temperature within the range of 20° to 250°C. It is preferred that the reaction be carried out at a temperature within the range of about 80° to 230°C over a period of from about 3 to 5 hours.

Particularly when the present amide derivatives are used in mineral oils the preparation of the amides also may be carried out in said mineral oil. The reaction temperature generally varies between 100° and 200°C. During the reaction, which usually lasts 3 to 7 hours, nitrogen is passed through. After cooling and filtration generally a clear solution is obtained. When the amide derivatives of the present invention are used in lubricant compositions often also metal salts of carboxylic acids are incorporated into these compositions. The molar ratio of amide to metal soap will as a rule be about 1:1 then.

In that case it is recommended to use as starting material the anhydrides of the carboxylic acids according to the first-mentioned formula, to amidate these and subsequently to react the reaction

product with a metal salt or metal oxide. In this last-mentioned case the reaction is advantageously carried out in the presence of a small amount of water of up to 2,5% by weight of the reaction mixture. Suitable metals from which the oxides or salts may be derived are: alkali metals, alkaline earth metals, zinc, cadmium and/or lead. Instead of the salts or oxides of said metals hydroxides, carbonates or lower alcoholates may be used. The resulting reaction mixture is heated to a temperature in the range of 20° to 250°C, and preferably 80° to 230°C.

It will be obvious that besides the carboxylic acids that correspond to the above-mentioned structural formula carboxylic acids having a somewhat different structure may be present. The proportion in which they may be present is governed on the one hand by technical price and production factors and on the other by the demands made on the endproduct.

Thus, in the preparation of the acids according to the first-mentioned formula also other branched and unbranched acids are obtained. Particularly the presence of the branched acids of the formula

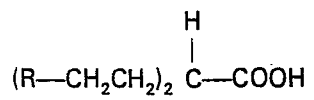

and/or

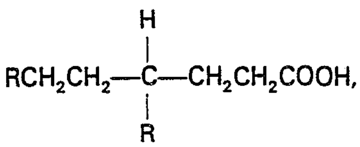

where R has the same meaning as in the first-mentioned formula, is found to lead to amide derivatives which excellently mix with mineral oil fractions.

Instead of by the above preparation by a direct reaction of the amine with the acid, the anhydride or the ester the preparation may be carried out by first converting the acid in the acid chloride with, for instance, thionyl chloride or phosphorus trichloride or phosphorus pentachloride, after which the acid chloride is added to the amine in the presence of a base such as an aqueous sodium hydroxide solution or pyridine.

Another attractive method of preparing the amides of the branched-chain acids according to the first-mentioned formula is characterized by direct reaction of the acid or mixture of acids and the equivalent amount of 1 or more isocyanate groups-containing compounds or mixtures thereof. The use of isocyanate groups-containing compounds instead of the amino-groups containing compounds has the additional advantage that the reaction proceeds at a higher speed and as byproduct only the readily isolated carbon dioxide is formed.

An important advantage of the novel amide compounds according to the present invention over the known compounds described in, for instance, the German Patent Specification 2 460 235 inter alia consists in the higher solvent retention. Moreover, when incorporated into rubber or polymers, the novel products according to the invention will less readily exude therefrom.

Further, they are excellently suitable to be applied as paper sizing agent, and for increasing the dropping point of paraffin waxes and asphalt and decreasing the viscosity of adhesives. The isocyanates to be used in the preparation of the present compounds may be of an aliphatic or aromatic character. If few or no coloured products are desired, then it is preferred to use aliphatic isocyanates. Preference is further given to isocyanates of the general formula $A{-}R_1{-}NCO$, where $R_1$ represents a (cyclo) aliphatic hydrocarbon having at least 6 carbon atoms, a phenyl group or naphthyl group, which groups may be substituted or not with one or more lower alkyl groups having 1 to 8, and preferably 1 to 6 carbon atoms, lower alkoxy groups having 1 to 8, and preferably 1 to 6 carbon atoms, aryl, for instance phenyl, and halogen such as chlorine or bromine, and A represents a —NCO group, or a $-R_2-CH_2-R_3-NCO)_n\ R_4$ NCO group, where $R_2$ has the meaning of a simple bond or an aliphatic hydrocarbon group having 1 to 4 carbon atoms, n is equal to 0 or higher, and $R_3$ and $R_4$ may be the same or different and may or may not have the same meaning as $R_1$.

As examples of such diisocyanates may be mentioned:

2-ethylhexyl isocyanate, butyl isocyanate, stearyl isocyanate. As examples of diisocyanates which can be defined by the formula OCNRNCO, where R represents a divalent aliphatic, cycloaliphatic or aromatic group, may be mentioned:

hexamethylene diisocyanate;
dimethyl hexamethylene diisocyanate;
trimethyl hexamethylene diisocyanate;
metaxylylene diisocyanate;
paraxylylene diisocyanate;
tetramethylene diisocyanate.

In the case where R represents an aromatic group, it may be substituted with a halogen, a lower alkyl or a lower alkoxy group.

As examples of such diisocyanates may be mentioned:

1-chloro-2,4-phenylene diisocyanate;
2,4-toluene diisocyanate;
a mixture of 2,4-toluene diisocyanate and 2,6-toluene diisocyanate;
tetramethylphenylene diisocyanate;

diphenylmethane-4,4'-diisocyanate;
metaphenylene diisocyanate;
paraphenylene diisocyanate;
1,5-naphthalene diisocyanate;
biphenyl-4,4'-diisocyanate;
diphenylmethane-4,4'-diisocyanate;
4,4'-isopropylidene diphenyl isocyanate;
benzophenone-4,4'-diisocyanate;
diphenylether diisocyanate or diphenylsulphide diisocyanate;
3,3'-dimethyldiphenyl-4,4'-diisocyanate;
3,3'-dimethoxydiphenyl-4,4'-diisocyanate;
3,3'-dichlorodiphenyl-4,4'-diisocyanate;
benzofuran-2,7-diisocyanate.

Examples of diisocyanates having a cycloaliphatic group include isophoron diisocyanate, dicyclohexyl methane diisocyanate and 1,4-cyclohexane diisocyanate.

The temperature at which the reaction takes place between the monocarboxylic acid and the isocyanate should be established experimentally. It will generally be in the range of 40° to 250°C. The reaction can be followed by the amount of carbon dioxide evolved in the process. The above-described amides are especially suitable to be incorporated as lubricants in styrene/acrylonitrile/butadiene copolymers.

By styrene/acrylonitrile/butadiene (ABS) copolymers are to be understood here the conventional ABS plastics which are composed of styrene-acrylonitrile copolymers as a continuous phase and a dispersed phase of butadiene-acrylonitrile rubber, or a butadiene-containing rubber onto which styrene-acrylonitrile monomers are grafted.

The lubricants may be incorporated into the ABS copolymers in the usual manner. This may be done by milling at temperatures between 130° and 160°C. The lubricant may be added along with other ingredients, or separately. The above amides are further suitable to be used — along with the other derivatives of the present invention — in polyvinyl chloride or copolymers of polyvinyl chloride. By polyvinyl chloride and copolymers of polyvinyl chloride are to be understood here all possible types of homopolymers of vinyl chloride and post-chlorinated polyvinyl chloride, and also copolymers with vinyl chloride as the most important constituent and a small amount of other copolymerizable monomers, such as copolymers of vinyl chloride and vinyl acetate, copolymers of vinyl chloride and vinylidene chloride, copolymers of vinyl chloride and acrylonitrile, copolymers of vinyl chloride and maleic or fumaric esters and copolymers of vinyl chloride and styrene, and also mixtures containing a high percentage of polyvinyl chloride resin and a small percentage of some other synthetic resin, such as chlorinated polyethylene, copolymers of acrylonitrile, butadiene and styrene.

The lubricants may be incorporated into the polyvinyl chloride or copolymers thereof in the usual manner. This may be done by mixing on a roll or in a mixer of the Banbury type. Alternatively, the lubricant may be dissolved or dispersed in some appropriate solvent. The lubricant may be added along with other composition ingredients such as stabilizers, fillers and plasticizers, or in a separate step. The physical properties of the formulated resin composition may be considerably varied by changing the amount and the nature of the constituents to be incorporated therein without detracting from the lubricating properties of the present lubricants.

For a man skilled in the art it will generally not be difficult to find the most suitable percentage for obtaining an optimum effect for each use envisaged. In a number of cases it will be possible for the derivatives according to the present invention to be used as such, i.e. without admixing other known polymer and/or engine lubricating agents, but generally it will be preferred that other products should be admixed in order to obtain more favourable physical and/or chemical properties. In this connection special mention should be made of wax compositions that substantially consist of a mixture of branched-chain or straight-chain acids and derivatives according to the present invention.

The invention is further described in, but not limited by the following examples.

## Example I

A slurry made up of 17,4 g of manganese (III) acetate in 100 ml of acetic anhydride and 84 g (0,6 moles) of 1-decene was slowly added, with stirring, over a period of 1,5 to 5 hours in an atmosphere of nitrogen, to a previously provided mixture of 900 ml (9,5 moles) of acetic anhydride and 42 g (0,3 moles) of 1-decene. The reaction temperature was 125°C, and the stirring speed 800 revolutions per minute. Upon completion of the reaction the mixture was cooled to room temperature and filtered to remove the manganese (II) acetate formed. Subsequently, acetic anhydride, acetic acid formed and unconverted 1-decene were distilled off. To the residue there were added 200 ml of acetic acid and 25 ml of water and the mixture was heated for 1 hour to 100°C, with vigorous stirring. Then the acetic acid-water mixture was distilled off, followed by removing the $R_1$ fraction (=lauric acid) in a rotary vacuum evaporator of the Leybold KDL-4 type under the following conditions:

| | |
|---|---|
| Sleeve temperature | 118°C |
| cold finger | 25°C |
| metering vessel | 40°C |
| pressure | $10^{-3}$mm Hg |
| feed rate | 400 ml/hour |

After distillation the telomeric acids had the following compositional analysis:

| | weight % |
|---|---|
| $R_1$ | 0,3 |
| $R_2$ | 19,8 |
| $R_3$ | 33,6 |
| $R_4$ | 21,5 |
| $R_5$ | 15,9 |
| $R \geq 6$ | 8,8 |

The acid number of the heavy fraction was found to be 109,3.

75 g of n-butanol and 50 g of the resulting telomeric acids were intermixed, after which 1 g of manganese (II) acetate was added to the mixture. After 4 hours heating at 280°C in an autoclave under nitrogen the reaction mixture was cooled and the excess of n-butanol distilled off. The residue was introduced into ether, after which the catalyst was washed out with water. Of the resulting ester mixture the most important properties, such as the viscosity at different temperatures and the pour point (in conformity with ASTM D 97—66), were determined and compared with those of a few lubricating oils and/or the base fluids thereof. The results are given in the following table.

A = esters prepared as described in the above example.

B = commercially available engine oil, between 80 and 90% by weight of which is formed by the base fluid $B_1$.

$B_1$ = a base fluid of B (a paraffinic mineral oil of the type often used in engine oils),

C = an engine oil whose base fluid is composed of 70% by weight of hydrogenated oligo $\alpha$-olefine and 30% by weight of ester.

D = a fully synthetic engine oil whose composition is similar to that of C.

E = a base fluid for C and D, and consisting of trimethylol propane esters of $C_8$–$C_{10}$ acids.

F = hydrogenated oligo $\alpha$-olefins.

| Type | Viscosity in mm²/s at | | | Viscosity index ($VI_E$) | Pour Point °C |
|---|---|---|---|---|---|
| | 25°C | 40°C | 100°C | | |
| A | 32,2 | 18,8 | 5,03 | 216 | <–60 |
| B | 331 | 156 | 19,5 | 141 | –20 |
| $B_1$ | 63,0 | 32,0 | 5,14 | 83 | –13 |
| C | 245 | 130 | 22,5 | 220 | –45 |
| D | 74,0 | 38,1 | 6,79 | 146 | –55 |
| E | 38,0 | 21,2 | 4,58 | 134 | –45 |
| F | 53,9 | 30,0 | 6,46 | – | <–60 |

From the above table it is evident that as far as the properties mentioned in it are concerned the esters according to the invention can compete with the best commercially available base fluids for lubricating oils. Also a comparative test for thermal oxidative behaviour at 200°C gives a favourable picture for the esters according to the invention.

The last-mentioned test was carried out as follows.

3 g of oil were heated in an aluminium lid 6 cm in diameter and 1 cm in height for 16 and 48 hours at 200°C while air was passed through. The consistency of the oil, the colour (1=water white, 10=black) and the percentage evaporated material (% loss of weight) were evaluated. The results are given in the following table.

| Type | Evaluation after 16 hours | | | Evaluation after 48 hours | | |
|---|---|---|---|---|---|---|
| | consistency | colour | % loss of weight | consistency | colour | % loss of weight |
| A | oil | 7 | 30 | viscous oil | 9 | 47 |
| B | oil | 9 | 36 | pasty | 10 | 43 |
| $B_1$ | oil | 10 | 34 | pasty | 10 | 61 |
| C | oil | 7 | 14 | viscous oil | 9 | 43 |
| D | oil | 6 | 17 | viscous oil | 8–9 | 43 |
| E | oil | 4 | 34 | pasty | 6 | 63 |
| F | oil | 5 | 27 | viscous oil | 8 | 47 |

## Example II

In a similar way as indicated in Example I a mixture of telomeric acids was prepared starting from 1-decene, but in such a way understanding that a greater part of the 1-decene had previously been charged to the reaction vessel.

A slurry made up of 33 g of manganese (III) acetate in 270 g of acetic anhydride and 25 g of acetic acid and 220 g of 1-decene was slowly added, with stirring, over a period of 7,2 hours in an atmosphere of nitrogen, to a previously provided mixture of 300 g acetic anhydride, 28 g acetic acid and 140 g of 1-decene. Upon completion of the reaction the mixture was filtered. Subsequently, acetic anhydride, acetic acid and unconverted 1-decene were distilled off (80°C, 19 mbar). To the residue there were added 160 ml of acetic acid and 40 ml of water and the mixture was heated for 3 hours with refluxing. Subsequently, the acetic acid-water mixture was distilled off. (100°C, 130 mbar). The composition of the mixture thus obtained was as follows (according to a GPC analysis):

| | weight % |
|---|---|
| $R_1$ | 7 (lauric acid) |
| $R_2$ | 16 |
| $R_3$ | 20 |
| $R_4$ | 16 |
| $R_5$ | 26 |
| $R \geqslant 6$ | 15 |

Subsequently, the reaction mixture was distilled in a rotary vacuum evaporator of the Leybold KDL-4 type under the following conditions:

| | |
|---|---|
| sleeve temperature | 140°C |
| cold finger | 45°C |
| metering vessel | 25°C |
| pressure | 0,05 k Pa |
| feed rate | 295 g/hour |

After distillation the telomeric acids had the following compositional analysis:

| | weight % |
|---|---|
| $R_1$ | <1 |
| $R_2$ | 6 |
| $R_3$ | 24 |
| $R_4$ | 22 |
| $R_5$ | 31 |
| R>6 | 17 |

The acid number of the acid fraction was found to be 77.

## Example III

In a similar way as indicated in Example I a number of butanol esters were prepared starting from 1-hexene, 1-octene, 1-decene and dodecene as $\alpha$-olefin.

The results of the measured viscosity behaviour and the pour point values are given in the following table:

A = esters derived from 1-hexene ($C_6$)

B = esters derived from 1-octene ($C_8$)

C = esters derived from 1-decene ($C_{10}$)

D = esters derived from 1-dodecene ($C_{12}$)

| Type | Viscosity in $mm^2/s$ at | | | Viscosity index ($VI_E$) | Pour Point °C |
|---|---|---|---|---|---|
| | 25°C | 40°C | 100°C | | |
| A | 11,4 | 7,1 | 2,07 | 72 | <−65 |
| B | 17,8 | 11,3 | 2,94 | 122 | <−60 |
| C | 32,2 | 18,8 | 5,03 | 216 | <−60 |
| D | 39,3 | 22,5 | 4,94 | 152 | −32 |

With a monocarboxylic acid obtained by reacting one $\alpha$-olefin with acetic acid being indicated by $R_1$, a monocarboxylic acid obtained by reacting two $\alpha$-olefins by $R_2$, a monocarboxylic acid by reacting three $\alpha$-olefins by $R_3$, etc., the following product distributions (in wt %) can be given for the above ester mixtures A, B, C and D:

| | A | B | C | D |
|---|---|---|---|---|
| $R_1$ | — | 0,8 | 0,3 | — |
| $R_2$ | 29,2 | 28,5 | 18,6 | 31,0 |
| $R_3$ | 33,9 | 32,0 | 33,7 | 32,3 |
| $R_4$ | 19,3 | 19,1 | 22,0 | 19,9 |
| $R_5$ | 12,1 | 13,3 | 16,4 | 11,5 |
| R⩾6 | 5,6 | 6,4 | 9,0 | 5,4 |

Example IV

In a similar way as indicated in Example I and after removal of the $R_1$ fraction neopentyl glycol esters were prepared from branched-chain acids derived from 1-decene.

On a sample of the resulting esters the following properties were measured:

| Viscosity in mm²/s at | | | Viscosity index $VI_E$ | Pour Point °C |
|---|---|---|---|---|
| 25°C | 40°C | 100°C | | |
| 45,5 | 24,7 | 4,80 | 115 | –47 |

Example V

In a similar way as described in Example I, but without removing the $R_1$ fraction, trimethylol propane esters were prepared from the mixture of branched-chain acids and lauric acid derived from 1-decene.

The product distribution of the mixture of acids used for preparing the present esters was as follows:

| | wt% |
|---|---|
| $R_1$ | 23,6 |
| $R_2$ | 20,9 |
| $R_3$ | 23,5 |
| $R_4$ | 17,8 |
| $R \geqslant 5$ | 14,2 |

On a sample of the resulting esters the following properties were measured:

| Viscosity in mm²/s at | | | Viscosity index $VI_E$ | Pour Point °C |
|---|---|---|---|---|
| 25°C | 40°C | 100°C | | |
| 93,6 | 48,4 | 8,57 | 153 | –25 |

Example VI

Using the same procedure as described in Example I 1-hexene was used to prepare a mixture of telomeric acids from which the $R_1$ fraction was removed by distillation.

To 250 g (712 meq) of these telomeric acids 2,25 g (7 mmoles) of tetrabutyl ammonium bromide were added, after which the mixture was heated to 180°C. At this temperature and over a period of 10 minutes 132,7 g (712 mmoles) of 1,2-epoxydodecane were added, after which the reaction mixture was heated until all of the acid had been converted. After the reaction mixture had been cooled to 70°C, 200 ml of toluene were added and washed three times with 100 ml of water to remove the catalyst. The solvent was removed in a rotary vacuum evaporator.

374 g of the resulting $\beta$-hydroxy ester (573 meq) were mixed with 79,1 g (602 meq) n-heptanoic acid and heated to 230°C.

Subsequently, xylene was slowly added through the reflux condenser until the reaction mixture gently refluxed. The reaction was stopped when no longer any water evolved (after about 20 hours). Solvent and excess acid were removed by distillation, the reaction product being left in the flask. The results of the measured viscosity behaviour and the pour points are summarized in the table below:

A = esters derived from telomeric acids based on 1-hexane, 1,2-epoxydodecane and n-heptanoic acid;

B = esters derived from the telomeric acids of Example II, propylene oxide and n-heptanoic acid;

C = esters derived from telomeric acids of Example I, epoxidized 2-ethylhexyl oleate and lauric acid.

| Type | Acid number mg KOH/g | Viscosity in mm²/s at | | pour point °C | Visc. Ind. $VI_E$ | % evaporated after 200°C | |
|---|---|---|---|---|---|---|---|
| | | 40°C | 100°C | | | 16 h | 48 h |
| A | 3,5 | 32,7 | 5,97 | −42 | 129 | 57% | 69% |
| B | <0.1 | 66,8 | 10,17 | −39 | 138 | 28% | 46% |
| C | 0,4 | 89,8 | 12,65 | ≪−23 | 138 | 21% | 36% |

Example VII

The following example illustrates the fitness of the derivatives according to the present invention as lubricating agent in polymers.

The butanol ester mixture prepared in Example I, of which the product distribution was as follows:

| | wt % |
|---|---|
| $R_1$ | 0,3 |
| $R_2$ | 18,6 |
| $R_3$ | 33,7 |
| $R_4$ | 22,0 |
| $R_5$ | 16,4 |
| $R\geqslant 6$ | 9,0 |

was incorporated into a hard polyvinyl chloride (PVC) formulation and compared with a few commercially available lubricants.

Product A=butane diol ester of montanic acid of which 40% is saponified with calcium.
Product B=a mixture composed of tridecyl stearate and equal parts by weight of glycerol monooleate and pentaerythritol adipate/oleate
Product C=glycerol mono-ricinoleate (a distinct internal lubricant)
Product D=the above-mentioned butanol ester mixture

Use was made of the following test methods:

1. *Brabender test* for determining the rheological properties, the most important parameters being the gelation time, and the melt viscosity (torque upon gelation and the torque 5 minutes after gelation).
2. *High speed mill test* for studying the behaviour during processing, such as calendering. The polymer is observed for sticking to the roll (stick time) and change in colour both at elevated temperature and at high speeds.
3. *Clarity.* This property was determined by moulding formulated PVC mixtures into plates about 1 mm thick and subsequently visually evaluating the clarity.

*Procedure*

Each formulation was intensively mixed on a Papenmeier mixer. Part of the mixture was used for testing in the Brabender Plasticorder under the following conditions:

| | |
|---|---|
| temperature | 170°C |
| speed | 30 revolutions per minute |
| sample weight | 30 g |

Another part of the mixture was thoroughly mixed on a roll mill at 160°C until the mixture was entirely homogeneous. The required samples were cut out of a rolled sheet about 2 mm thick.

The samples were heated in an air circulation oven at 185°C, from which they were removed at 10 minute intervals, after which they were visually evaluated for change in colour. This colour change was taken as a measure of the decomposition rate of the PVC compound. The results of the experiments are combined in the following table and rated from 1 to 5, where

3=slow gradual change;
4=good early colour, rapid colour change;
5=good
1=poor
2=fairly rapid gradual degradation.

The formulation of the polyvinyl chloride used in this example was as follows:

| | parts by weight | | | |
|---|---|---|---|---|
| PVC-suspension polymer | 100 | | | |
| dibutyl tin-bis-lauryl mercaptan | 1,0 | | | |
| mixture of monobutyl tin-tris-isooctylthio glycolate, dibutyl tin bis isooctyl thio-glycolate, epoxidized soy bean oil, phenolic antioxidant | 1,0 | | | |
| lubricant | 0,1 | 0,3 | 0,5 | 1,0 |

1. Brabender test

| lubricant (parts by weight) / type of lubricant | 0,1 | 0,3 | 0,5 | 1,0 |
|---|---|---|---|---|
| | gelation time(s) | | | |
| control | 85 | 85 | 85 | 85 |
| product A | 100 | 270 | 330 | 890 |
| product B | 60 | 70 | 105 | 110 |
| product C | 60 | 55 | 55 | 55 |
| product D (invention) | 50 | 130 | 190 | – |
| | torque upon gelation | | | |
| control | 650 | 650 | 650 | 650 |
| product A | 640 | 570 | 540 | 480 |
| product B | 660 | 630 | 610 | 600 |
| product C | 640 | 660 | 600 | 530 |
| product D (invention) | 620 | 600 | 600 | – |

2. High speed mill test

| Lubricant (parts by weight) / type of lubricant | 0,1 | 0,3 | 0,5 |
|---|---|---|---|
| | time (min) after which polymer sticks to roll | | |
| control | 10 | 10 | 10 |
| product A | 35 | 40 | 40 |
| product B | 15 | 35 | 35 |
| product C | – | 10 | 5 |
| product D | 10 | 5 | 15 |

The above test results show that the product D according to the invention may best be considered an internal lubricant. This was also confirmed by the test for clarity and the oven test.

3. Clarity and thermal stability

| lubricant / type of lubricant | 0,1 | 0,3 | 0,5 | 0,1 | 0,3 | 0,5 |
|---|---|---|---|---|---|---|
| | clarity | | | thermal stability | | |
| control | good | good | good | 3 | 3 | 3 |
| product A | excellent | poor | poor | 3 | 4 | 3 |
| product B | good | poor | – | 3 | 4 | 4 |
| product C | good | good | poor | 3 | 4 | 3 |
| product D | excellent | excellent | good | 3 | 4 | 3 |

Example VIII

In this example a number of commercial lubricants for PVC are compared with the ethylene glycol-ester mixture of branched-chain monocarboxylic acids according to the invention, where n represents an integer of from 19 to 23. The formulation of the PVC used in this example was as follows:

| | parts by weight |
|---|---|
| PVC suspension polymer | 100 |
| dibutyl tin bisisooctyl thioglycolate | 2 |
| lubricant | 0,1 0,3 0,5 |

The test programme was carried out as indicated in Example VII

Product A = glycerol monoricinoleate

Product B = paraffin wax

Product C = 1,3-butane diol ester of montanic acid, 40% by weight of it being saponified with calcium.

Product D = the ethylene glycol ester mixture according to the invention.

1. Brabender test

| type of lubricant | parts by weight | gelation time (s) | torque on gelation | torque 5 min after gelation |
|---|---|---|---|---|
| control | 0 | 85 | 650 | 450 |
| product A | 0,5 | 55 | 650 | 450 |
| product B | 0,5 | 185 | 600 | 450 |
| product C | 0,1 | 100 | 640 | 460 |
| | 0,3 | 270 | 570 | 460 |
| | 0,5 | 330 | 540 | 460 |
| product D (according to invention) | 0,5 | 160 | 610 | 450 |

2. High speed mill test and clarity

| type of lubricant | parts by weight | time (min) after which polymer sticks to roll | clarity 2 mm thick PVC–slice |
|---|---|---|---|
| control | 0 | 10 | excellent |
| product A | 0,5 | 5 | good |
| product B | 0,5 | 10 | moderate |
| product C | 0,1 | 35 | good |
| | 0,3 | >40 | moderate |
| | 0,5 | >40 | moderate |
| product D (according to invention) | 0,5 | 25 | good |

Example IX

In this example the results are given of tests on a number of ethylene glycol esters of branched monocarboxylic acids derived from hexene, octene, decene and dodecene.

The formulation of the PVC used was follows:

| | parts by weight |
|---|---|
| PVC-suspension polymer | 100 |
| di(β-carbobutoxyethyl)tin bisiso octylthioglycolate | 2 |
| lubricant | 0,3—1,0 |

Each formulation was intensively mixed on a Papenmeier mixer. Part of the mixture was used for testing in the Brabender Plasticorder under the following conditions:

| | |
|---|---|
| temperature | 160°C |
| speed | 30 revolutions per minute |
| sample weight | 32,5 g |
| pressure | 5 kg |

*Clarity*

To determine the influence of the lubricant on the clarity of the stabilized formulations 3 mm thick sheets had been pressed at 190°C. Samples of the gelation experiments were taken 10 minutes after gelation. The transmission of these sheets was measured with a Bausch and Lomb spectrophotometer. The transmission at 690 nm was used as a measure of the clarity of the sheet.

The product distribution of the mixtures used is given in the table below. A mixture of telomeric acids based on octene is referred to as TP8, TP standing for *t*otal *p*roduct, i.e. the mixture of telomeric acids as it is obtained without the n=1 fraction having been removed. The TP-products based on hexene (TP6), octene (TP8), decene (TP10) and dodecene (TP12) always have practically the same composition:

| | wt % |
|---|---|
| n=1 | 30—40 |
| n=2 | 15—20 |
| n=3 | 20—25 |
| n=4 | 12—15 |
| n=5 | 8—10 |
| n⩾6 | 4 — 6 |

Subjecting the TP fractions to distillation leads to a pure n=1 fraction along with an LTA (low telomeric acid fraction) and an HTA (heavy telomeric acid fraction). The weight distribution of these fractions is given in the table below.

| | wt % | | | | | |
|---|---|---|---|---|---|---|
| telomeric acid fraction | LTA 6 | LTA 8 | LTA 10 | HTA 6 | HTA 8 | HTA 10 |
| n=1 | <5 | <5 | <5 | <5 | <5 | <5 |
| 2 | >60 | >70 | >80 | 10 | 20 | 30 |
| 3 | <35 | <25 | <15 | 25 | 20 | 15 |
| 4 | | | | 25 | 20 | 15 |
| 5 | | | | 25 | 15 | 10 |
| 6 | | | | 10 | 10 | 5 |

The following table gives the results of the Brabender gelation tests and the clarity tests on ethylene glycol esters from the above-mentioned mixtures of telomeric acids.

| ester of ethylene glycol and | phr* | Brabender gelation tests | | | | | clarity of 3mm thick pressed sheet at 690 ppm % T |
|---|---|---|---|---|---|---|---|
| | | gelation time (min.) | fusion torque (m grams) | torque 10 min. after gelation (m grams) | temp. at fusion °C | temp. after 10 min. °C | |
| $HTA_6$ | 0,5 | 2,5 | 2600 | 2900 | 160 | 171 | 88 |
| | 0,68 | 3,0 | 2575 | 2900 | 159 | 170 | 87 |
| | 1.0 | 4,0 | 2450 | 2850 | 159 | 170 | 88 |
| $TP_8$ | 0,5 | 3,0 | 2500 | 2850 | 160 | 170 | 87 |
| | 0,68 | 3,1 | 2500 | 2900 | 158 | 169 | 88 |
| | 1,0 | 5,1 | 2500 | 2900 | 159 | 169 | 87 |
| $TP_{12}$ | 0,5 | 4,1 | 2500 | 2850 | 161 | 171 | 87 |
| | 0,68 | 5,5 | 2400 | 2850 | 160 | 169 | 67 |
| | 1,0 | 21,2 | 2500 | 2850 | 164 | 171 | 36 |
| $HTA_8$ | 0,5 | 2,4 | 2625 | 3000 | 154 | 168 | 81 |
| | 0,75 | 3,4 | 2550 | 2950 | 155 | 168 | 80 |
| | 1.0 | 8,6 | 2650 | 2950 | 159 | 168 | 71 |
| $HTA_{10}$ | 0.3 | 3,3 | 2575 | 2925 | 155 | 167 | 83 |
| | 0,5 | 7,5 | 2575 | 2950 | 158 | 167 | 61 |
| | 0,6 | 12,1 | 2450 | 2800 | 165 | 170 | 44 |
| | 0,75 | ±24 | 2600 | 2900 | 164 | 167 | 28 |
| ethylene glycol ester of montanic acid | 0,3 | 2,7 | 2600 | 2950 | 150 | 169 | 83 |
| | 0,5 | 6,3 | 2575 | 2900 | 163 | 171 | 69 |
| | 0,75 | 21,8 | 2600 | 2900 | 165 | 170 | 41 |
| control | 0 | 1,2 | 2700 | 2850 | 156 | 171 | 85 |

*phr = parts per hundred resin.

From the above table it is evident that the external effect of the liquid ethylene glycol esters according to the invention increases with increasing length of the olefin from which the mixture of telomeric acids is derived.

Example X

In this example it is demonstrated that also esters derived from the $HTA_8$ described in Example IX, an epoxide from an $\alpha$-olefin having 15 to 18 carbon atoms and a straight-chain carboxylic acid such as lauric acid are very suitable to be used as a lubricant in PVC. The diester was prepared in the same manner as described in Example VI. Also tested as lubricant were the diester of ethylene oxide, $HTA_{10}$ and stearic acid, the diester of propylene oxide, $HTA_{10}$ and n-heptanoic acid, the diester of epoxidized 2-ethylhexyl oleate, $HTA_{10}$ and lauric acid and the diester of epoxidized 2-ethylhexyl oleate and the anhydride of $TP_{10}$. The formulation of the PVC used was entirely identical with the one of Example VIII.

The results are given in the table below.

| type of lubricant ester derived from: | phr | Brabender gelation tests: gelation time (min.) | fusion torque (m. grams) | torque 10 min. after gelation (m. grams) | temp. at fusion (°C) | temp. after 10 min. (°C) | clarity of 3 mm thick pressed sheet at 690 nm % T |
|---|---|---|---|---|---|---|---|
| $C_{15}-C_{18}$ epoxide $HTA_8$ and lauric acid | 0,3 | 4,5 | 2500 | 2775 | 162 | 170 | 84 |
| | 0,5 | 8,4 | 2450 | 2800 | 161 | 169 | 63 |
| | 0,6 | 11,1 | 2450 | 2800 | 164 | 171 | 54 |
| ethylene oxide, $HTA_{10}$ and stearic acid | 0,3 | 3,8 | 2450 | 2825 | 162 | 171 | 81 |
| | 0,5 | 6,5 | 2500 | 2850 | 162 | 169 | 73 |
| | 0,6 | 10,2 | 2500 | 2775 | 165 | 170 | 57 |
| propylene oxide $HTA_{10}$ and n-heptanoic acid | 0,3 | 3,9 | 2500 | 2850 | 160 | 169 | 83 |
| | 0,5 | 9,4 | 2525 | 2825 | 163 | 169 | 63 |
| epoxidized 2-ethylhexyl oleate, $HTA_{10}$ and lauric acid | 0,3 | 2,4 | 2600 | 2850 | 158 | 169 | 86 |
| | 0,5 | 3,1 | 2500 | 2875 | 159 | 169 | 83 |
| epoxidized 2-ethylhexyl oleate and the anhydride of $TP_{10}$ | 0,3 | 2,4 | 2600 | 2900 | 156 | 171 | 83 |
| | 0,5 | 5,7 | 2500 | 2900 | 163 | 171 | 70 |

Example XI

In this example it is shown that also the esters of trimethylol propane and telomeric acids are excellently suitable to be used as lubricants in PVC. The formulation of the PVC used was again the same as the one used in Example IX. The results are summarized in the table below

| trimethylol propane and: | phr* | Brabender gelation tests: gelation time (min.) | fusion torque (m grams) | torque 10 min. after gelation (m grams) | temp. at fusion °C | temp. after 10 min. °C | clarity of 3mm thick pressed sheet at 690 nm % T |
|---|---|---|---|---|---|---|---|
| $TP_{12}$ | 0,3 | 3,0 | 2450 | 2900 | 159 | 170 | 85 |
| | 0,5 | 6,6 | 2500 | 2850 | 163 | 170 | 66 |
| | 0,68 | 11,9 | 2450 | 2850 | 163 | 170 | 50 |
| $HTA_6$ | 0,5 | 3,2 | 2550 | 2875 | 161 | 171 | 87 |
| | 0,68 | 3,3 | 2450 | 2850 | 160 | 170 | 87 |
| | 1,0 | 6,5 | 2475 | 2850 | 163 | 170 | 87 |
| $HTA_8$ | 0,3 | 3,3 | 2500 | 2950 | 154 | 167 | 78 |
| | 0,5 | 6,5 | 2500 | 2900 | 157 | 167 | 69 |
| | 0,6 | 10,2 | 2475 | 2850 | 164 | 170 | 54 |
| $HTA_{10}$ | 0,1 | 2,8 | 2600 | 2850 | 160 | 170 | 88 |
| | 0,3 | 6,8 | 2500 | 2850 | 163 | 170 | 67 |
| | 0,5 | 16,0 | 2500 | 2850 | 167 | 171 | 60 |

Example XII

For the preparation of branched-chain acids use was made of a commercially available starting mixture of olefins consisting of about 22% by weight of olefins having not more than 28 carbon atoms and about 78% by weight of olefins having at least 30 carbon atoms ($C_{30}$+olefins), about 66% by weight of the olefins being $\alpha$-olefins. The remaining olefin compounds were vinylidene compounds. The reaction was carried out in a stirred (700 r.p.m.) reactor provided with 8 baffles and equipped with a stirrer having 6 diametrically opposed blades. Into this reaction vessel there were charged 12,5 litres (132 moles) of acetic anhydride. The liquid was heated to 120°C while nitrogen was slowly passed through to remove the oxygen present in it. With the liquid being kept at 120°C, first of all the $C_{30+}$ olefin mixture was added. Of this mixture in all 1175 g (2,5 moles) were added over a period of 210 minutes. 12 minutes after a start had been made with adding olefin a slurry of 0,625 moles Mn (III) acetate in 2,5 l acetic anhydride was added over a period of 216 minutes (so for a period of 18 minutes after the last of the olefin had been added addition of Mn (III) acetate was continued to ensure complete conversion of the olefin).

The mixture was subsequently filtered to remove the Mn (II) acetate that had formed. Next, acetic anhydride and the acetic acid formed were removed by distillation. To the residue there were added 2,5 l acetic acid and 0,3 l water. With vigorous stirring the mixture was boiled with refluxing to hydrolyse the obtained anhydrides. Finally, the water-acetic acid layer was separated off, the product washed 3 times with hot water and dried.

The resulting mixture of straight-chain and branched-chain acids had an acid number of 86 and was esterified under an atmosphere of nitrogen with equivalent amounts of suitable alcohols.

In the esterification with ethylene glycol the following procedure was used.

To 300 grammes of the above-described mixture of melted acids there were added 1,05 equivalents of ethylene glycol and 0,6 grammes of zinc acetate. The mixture was stirred at 190°C and the water evolved was carried off by a nitrogen stream. After 5 hours the remaining water and alcohol were removed under reduced pressure (14 mm Hg).

A similar procedure was used for the preparation of the ethylene glycol ester of a $C_{11-26+}$-acid. Further a mixture was prepared of the ethylene glycol ester of a $C_{30+}$ acid and the calcium soap thereof.

The resulting products were tested in a PVC formulation of the following composition:

PVC-suspension polymer 100

Tribasic lead sulphate 2

Lubricant 0,1—0,5

Each formulation was intensively mixed on a Papenmeier mixer. Part of the mixture was tested in the Brabender plasticorder under the following conditions:

temperature : 170°C
speed : 30 revolutions per minute
sample weight: 33,5 g
pressure : 5 kg

| Lubricant | concentration | gelation time (min.) | fusion torque (m grams) | temperature at fusion (°C) | torque 10 min. after gelation (m grams) | temperature 10 min. after gelation (°C) |
|---|---|---|---|---|---|---|
| ethylene glycol. ester $C_{30}$+ acid | 0,1 | 3,4 | 2400 | 164 | 2600 | 175 |
| | 0,3 | 9,2 | 2200 | 165 | 2500 | 174 |
| | 0,5 | >20 | – | – | – | – |
| ethylene glycol $C_{22-26+}$ acid | 0,1 | <1 | – | – | 2600 | 173 |
| | 0,3 | 3,1 | 2200 | 162 | 2625 | 174 |
| | 0,5 | 4,0 | 2300 | 165 | 2600 | 174 |
| Ca-soap of $C_{30+}$ acid | 0,1 | 2,3 | 2300 | 160 | 2400 | 174 |
| | 0.3 | 14,8 | 2200 | 168 | 2300 | 173 |
| | 0,5 | >20 | – | – | – | – |
| 60% ethylene glycol ester $C_{30+}$ acid + 40% Ca-soap $C_{30+}$ acid | 0,1 | 2,0 | 2150 | 160 | 2500 | 173 |
| | 0,3 | 11,0 | 2100 | 170 | 2200 | 173 |
| | 0,5 | >20 | – | – | – | – |
| ethylene glycol ester of montanic acid | 0,1 | 2,2 | 2250 | 163 | 2500 | 175 |
| | 0,3 | 3,7 | 2300 | 165 | 2550 | 175 |
| | 0,5 | 5,8 | 2200 | 170 | 2400 | 175 |

In the above table it is clearly demonstrated that the above compounds according to the invention display an excellent external lubricating effect in a lead-stabilized system.

Example XIII

In this example it is demonstrated that the products according to the invention derived from telomeric acids derived from a $C_{30+}$ olefin mixture and a $C_{22-26}$ olefin mixture display an excellent external effect. The same PVC-formulation was used as indicated in Example IX. The results are given in the table below.

| type of lubricant | phr | Brabender gelation tests: gelation time (min.) | fusion torque (m. grams) | torque 10 min. after gelation (m. grams) | temp. at fusion (°C) | temp. after 10 min. (°C) | clarity of 3 mm thick pressed sheet at 690 nm % T |
|---|---|---|---|---|---|---|---|
| diester of polyethyleneoxide glycol (mol. weight 1000) and $C_{30+}$ acid | 0,3 | 3,9 | 2600 | 2900 | 162 | 171 | 80 |
| | 0,5 | 8,1 | 2600 | 2900 | 163 | 170 | 63 |
| diamide of hexamethylene diamine and $C_{30+}$ acid | 0,3 | 8,9 | 2600 | 2850 | 165 | 171 | 64 |
| | 0,5 | 12,7 | 2575 | 2825 | 167 | 171 | 33 |
| diamide of difenyl-* diamine methane and $C_{30+}$ acid | 0,3 | 5,0 | 2600 | 2850 | 162 | 171 | 80 |
| | 0,5 | 8,9 | 2600 | 2900 | 165 | 171 | 75 |
| diester of 4,4'-isopropylidene diphenoxydiethanol and $C_{22\ 26+}$ acid | 0,3 | 2,2 | 2575 | 2900 | 155 | 169 | 75 |
| | 0,5 | 3,3 | 2500 | 2900 | 159 | 169 | 71 |

*prepared from diphenylmethane-4,4'-diisocyanate (MDI)

Example XIV

In this example the fitness to be incorporated into wax compositions is illustrated for the present compounds according to the invention, where n is an integer from 17 to 42.

For the preparation of the esters and salts use was made of the same $C_{30+}$ acid mixture as exemplified in Example XII.

In the esterification with pentaerythritol the following procedure was used.

To 300 grammes of the above-described mixture of melted acids there were added 16,4 grammes of pentaerythritol (1,05 equivalents) and 0,6 grammes of zinc acetate. The mixture was stirred at 190°C and the water evolved was carried off by a nitrogen stream. After 5 hours the remaining water and alcohol were removed under reduced pressure (14 mm Hg). The solidification point of the resulting tetra ester was 77°C. The acid number was 10,3; the hydroxyl number was between 2 and 5. A similar procedure was used for the preparation of ethylene glycol ester and 1,3-butanediol ester. The 1,3-butanediol ester was mixed with 40% by weight calcium soap.

On the resulting products the following properties were measured:

1. *Ubbelohde dropping point °C* (heating rate 1°C/min.) which is a measure of the "melting point" of the wax composition. The dropping point was determined with a Mettler FP 53 tester.
2. *Ubbelohde dropping point °C* (heating rate 1°C/min.) as under 1, but after mixing 1 part of wax with 4 parts of paraffin.
3. *Penetration of paraffin mixture,* in accordance with ASTM D 1321—70. In this way an indication of the hardness increasing effect of the esters is obtained.
4. *Continental solid point,* in accordance with ASTM D 938—49. This point corresponds to the congealing point of 1 part of wax, 4 parts of paraffin and 15 parts of turpentine.
5. *Consistency of paste,* as described in "Vom Wachs, Hoechster Beiträge zur Kenntnis der Wachse", Farbw. Hoechst, Frankfurt-Hoechst, Vol. II, Beitrag II, p. 50—51. In that test a stamp of some particular weight and dimensions is slowly, while subjected to a gradually increasing pressure, brought into contact with the paste.
6. *Solvent retention,* to determine this property 1 part of the wax to be examined was mixed with 4 parts of paraffin and 15 parts of turpentine and the resulting mixture was brought into a tin. After the closed tin had been left for 17 hours at 23°C, it was placed in a ventilated oven. After it had been in the oven for 7 days at 32°C and subsequently on a table for another 7 days at 20°C, the solvent retention was measured. It is expressed as follows:

$$\text{solvent retention} = 100\% - \frac{\text{amount of turpentine lost}}{\text{original amount of turpentine}} \times 100\%$$

The results are listed in the following table and compared with those of the known wax compositions or components thereof.

| Wax | dropping point | dropping[a] point of paraffin mixture (°C) | penetration[a] of paraffin mixture (0,1 mm) |
|---|---|---|---|
| paraffin | 60 | (60) | 17 |
| camauba prime yellow[b] | 85 | 81 | 7 |
| montanic acids | 86 | 79 | 5 |
| montanic acid ethylene glycol ester | 83 | 77 | 7 |
| montanic acid 1,3-butanediol ester—40% calcium soap | 102 | 90 | 5 |
| ethylene glycol ester (d) | 93 | 78 | 7 |
| 1,3-butanediol ester-40% calcium soap (d) | 106 | 83 | 6 |
| pentaerythritol ester (d) | 86 | 72 | 5 |

a. Paraffin mixture: 1 part of wax + 4 parts of paraffin.

b. Carnauba wax, which is a natural wax, containing 80% of esters of acids—long-chain (≥ C24) and substituted or unsubstituted cinnamic acid — and long-chain (≥ C30) alcohols.

| Wax | paste consistency ($g/cm^2$) | continental[c] solid point (°C) | solvent retention (%) |
|---|---|---|---|
| Carnauba fatty gray | 1500 | 40 | 31 |
| montanic acids | 1200 | 40 | 20 |
| montanic acid ethylene glycol ester | 1100 | – | – |
| montanic acid 1,3-butanediol ester—40% calcium soap | 1350 | 40 | 51 |
| ethylene glycol ester (d) | 1150 | 39 | 75 |
| 1,3 butanediol ester—20% calcium soap (d) | 1300 | 40 | 73 |
| pentaerythritol ester (d) | 800 | 42 | 63 |
| pentaerythritol ester—40% calcium soap (d) | 1350 | 41 | 37 |

c. Paste composition: 1 part of wax, 4 parts of paraffin; 15 parts of turpentine.

d. Derived from straight-chain and branched-chain acids according to the invention.

Example XV

Using the same procedure as in the preceding examples the following compounds were tested for their being entirely or partially suitable to replace the known carnauba and/or montanic acid waxes.

| Wax | dropping point (°C) | dropping point paraffin mixture (°C) | penetration of paraffin mixture (0,1 mm) |
|---|---|---|---|
| ester of $C_{30+}$ acid and stearyl alcohol | 83 | 72 | 9,0 |
| ester of $C_{30+}$ acid and the $C_{30+}$ alcohol obtained from it by reduction | 94 | 80 | 7,5 |
| ester of $C_{30+}$ acid and polyethylene-glycol (mol wt 600) | 86 | 72 | 13,0 |
| ester of $C_{30+}$ acid and polyethylene glycol (mol. wt. 1000) | 86 | 73 | 13,0 |
| diester of $C_{22-26+}$ acid and 4,4'-isopropylidene diphenoxyethanol | 53 | 59 | 11,5 |
| diamide of $C_{22-26+}$ acid and 2,4-toluenediiso-cyanate | 111 | 92 | 8,0 |
| paraffin | 60 | (60) | 17 |

| Wax | continental solid point (°C) | paste consistency (g/cm²) | solvent retention (%) |
|---|---|---|---|
| ester of $C_{30+}$ acid and stearyl alcohol | 38 | 860 | 73 |
| ester of $C_{30+}$ acid and $C_{30+}$ alcohol | 45 | 950 | 63 |
| ester of $C_{30+}$ acid and polyethylene glycol (mol. wt. 600) | 43 | 1400 | 96 |
| ester of $C_{30+}$ acid and polyethylene glycol (mol. wt. 1000) | 39 | 1100 | 96 |

Example XVI

In this example it is demonstrated that the mixture of pentaerythritol esters prepared according to the procedure used in Example XIV can very well be emulsified. As emulgator there was used the combination oleic acid-amine. Examples of suitable amines include morpholine or 2-amino-2-methylpropanol. The emulsion was prepared by adding 5 grammes of morpholine to a melted mixture made up of 30 g of wax and 6 g of oleic acid at a temperature of 98°C and the mixture was stirred for 3/4 minutes. Subsequently, 20 g of boiling water were added over a period of 3/4 minutes while

increasing the stirring speed to 2000 revolutions per minute, which speed was maintained for 3 more minutes. Next, 180 g of boiling water were added over a period of 2 minutes, after which the mixture was rapidly cooled with an external ice bath and diluted with 60 g of cold water. The resulting emulsion was extraordinarily stable; the particle size was in the order of 0,5 μm.

Adding a polystyrene emulsion resulted in obtaining an excellent floor polish. The gloss obtained after drying bore comparison with that obtained after the use of a similar emulsion containing carnauba-prime-yellow as wax component to which no polystyrene emulsion had been added.

Example XVII

In the same way as indicated in Example XII for the esterification of a mixture of $C_{30+}$ acids, 12,7 g of hexamethylene diamine (1,05 equivalents) were added to 300 g of melted acids. The mixture was stirred at 240°C and the water evolved was carried off by a stream of nitrogen. After 5 hours the remaining volatile constituents were removed under reduced pressure (14 mm Hg). The acid number of the resulting reaction product was 4,2. In a similar way the amides were prepared from 4,4′-diaminodiphenylmethane and benzidine. The products were very hard, as is shown in the following table.

| Wax | acid number | Ubbelohde dropping point (°C) | Ubbelohde[a] dropping point paraffin mixture (°C) | Penetration[b] of paraffin mixture (0,1 mm) |
|---|---|---|---|---|
| paraffin | – | 60 | (60) | 17 |
| amide of hexamethylene diamine | 4,2 | 124 | 110 | 8 |
| amide of diaminodiphenyl methane | 9,8 | 128 | 100 | 6 |
| amide of benzidine | 10,4 | 165 | 103 | 6[c] |

a. paraffin mixture: 1 part of wax + 4 parts of paraffin.

b. in accordance with ASTM D 1321–70.

c. unreliable because of brittleness of sample.

Example XVIII

Using the same procedure as in Example XII 200 g of $C_{30+}$ acids (acid number 86,4) were esterified with 57 g of polypropylene glycol (average molecular weight 400) in the presence of 0,4 g of zinc acetate as catalyst. The esterification lasted 7 hours at 185°C and was carried out while passing through a stream of nitrogen. The resulting product had a dropping point of 73°C and could be emulsified to a milk white emulsion.

Example XIX

In a similar way as used in the preceding example 200 g of $C_{30+}$ acid were esterified with 92 g of polyethylene glycol (average molecular weight 600) in the presence of 0,4 g of zinc acetate. The resulting product was a hard ester wax having a dropping point of 89°C. It could readily be emulsified to a translucent wax emulsion.

Example XX

Starting from 1-octene a mixture of telomeric acids of the following weight distribution was prepared

| | wt % |
|---|---|
| n=1 | 1 |
| n=2 | 20 |
| n=3 | 33 |
| n=4 | 19 |
| n=5 | 14 |
| n=6 | 7 |

Into a three-neck flask there were charged 100 g of this mixture of telomeric acids having an acid number of 122 together with 41,15 g of tetraethylene pentamine, followed by heating to 230°C. The three-neck flask was provided with a Dean-Stark receiver. During the reaction $N_2$ was slowly passed through the mixture to remove the water formed. After 2 hours the reaction mixture was cooled. The product obtained was a low-viscous, light coloured liquid, which was dissolved in a mineral oil which has a viscosity ($\eta$ 40) at 40°C of 31,4 centistokes and at 100°C of 5,41 centistokes. The Pour Point of this oil was −11°C. Viscosity measurements and Pour Point determinations on solutions containing 10,3 and 0,5% by weight of the amide, respectively, gave the following results:

| Amide concentrations wt% | $\eta$ 40 C St | $\eta$ 100 C St | Pour Point °C |
|---|---|---|---|
| 0 | 31,4 | 5,41 | −11 |
| 10 | 36,6 | 5,68 | −11 |
| 3 | 32,9 | 5,49 | −10 |
| 0,5 | 31,5 | 5,34 | −13 |

Example XXI

Starting from 1-decene a mixture of telomeric acids of the following weight distribution was obtained:

| | wt% |
|---|---|
| n=1 | 3 |
| n=2 | 22 |
| n=3 | 30 |
| n=4 | 21 |
| n=5 | 15 |

The acid number of this telomeric acid mixture was 104. Of this mixture 100 g were mixed with 35,09 g of tetraethylene pentamine and heated to 230°C in the manner indicated in Example XX. The water removed with the aid of an $N_2$-stream was measured with a Dean-Stark receiver. After 2 hours the reaction was found to be terminated and the low-viscous reaction mixture was dissolved in a mineral oil of the same type as indicated in Example XX. The rheological properties of these solutions are given in the table below.

| Amide concentration wt % | $\eta$ 40 C St | $\eta$ 100 C St | Pour Point °C |
|---|---|---|---|
| 0 | 31,4 | 5,41 | −11 |
| 0,5 | 31,5 | 5,35 | −9 |
| 3 | 33,1 | 5,43 | −9 |
| 10 | 35,3 | 5,83 | −9 |

Example XXII

The procedure of example XXI was repeated, but in such a way that the n=1 fraction was not removed. The mixture of telomeric acids had the following composition.

| | wt % |
|---|---|
| n=1 | 32,1 |
| n=2 | 21,2 |
| n=3 | 21,9 |
| n=4 | 14,4 |
| n=5 | 10,3 |

The acid number of this mixture of carboxylic acids was 170. Of this mixture 98,43 g were mixed with 56,12 g of tetraethylene pentamine and again heated to 230°C in the same way as indicated in Example XXI. The resulting low viscous, light coloured liquid was dissolved in a mineral oil of the same type as mentioned in Example XXI. The rheological properties of these solutions are given in the table below.

| Amide concentration wt % | $\eta$ 40 C St | $\eta$ 100 C St | Pour Point °C |
|---|---|---|---|
| 0 | 31,4 | 5,41 | −11 |
| 0,5 | 31,0 | 5,36 | −9 |
| 3 | 31,6 | 5,37 | −8 |
| 10 | 33,5 | 5,49 | −12 |

Comparison with the results given in Example XXI clearly demonstrates that even a very high percentage of linear acid has practically no detrimental influence on the rheological properties of the oil.

Example XXIII

In this example the results are given of tests on amide derivatives of a telomeric acid mixture of Example II and tetraethylene pentamine (A) and pentaethylene hexamine (B), respectively. Further, the results are given of tests on the amide derivatives of tetraethylene pentamine and a TP (=total product) based on octadecene ($TP_{18}$)(C) and a heavy telomeric acid fraction ($HTA_{18}$) based on the same olefin (D), respectively. Viscosity measurements on solutions containing 3 and 10% by weight of the amide gave the following results:

| Amide | concentration | $\eta$ 40 C st | $\eta$ 100 C st |
|---|---|---|---|
| A | 3 | 32,3 | 5,36 |
| | 10 | 36,4 | 5,82 |
| B | 3 | 32,3 | 5,44 |
| | 10 | 34,9 | 5,70 |
| C | 3 | 32,0 | 5,42 |
| | 10 | 33,4 | 5,64 |
| D | 3 | 33,2 | 5,57 |
| | 10 | 36,9 | 6,07 |

Example XXIV

In this example it will be demonstrated that also the salts of the telomeric acids display a very remarkable viscosity behaviour in a mineral oil. The test procedure was similar to the one described in Example I. The base fluid was a mineral oil of the type often used in engine oils (a paraffinic commercial base fluid).

The telomeric acids used were based on decene (according to Example II) and $HTA_6$, $HTA_8$, $HTA_{10}$, $HTA_{14}$ and $HTA_{18}$ acid according to Example IX. The results are summarized in the following table below.

| Telomeric acid | metal | $\eta$ 40 (C st) | $\eta$ 100 (C st) | Pour point (°C) |
|---|---|---|---|---|
| Acid mixture of Example II | Ca | 36,16 | 5,98 | –12 |
| Acid mixture of Example II | Ba | 35,66 | 5,94 | –12 |
| Acid mixture of Example II | Li | 36,68 | 6,02 | –15 |
| $HTA_6$ | Ca | 37,98 | 6,52 | –11 |
| $HTA_8$ | Ca | 38,97 | 6,12 | –12 |
| $HTA_{10}$ | Ca | 38,93 | 6,17 | –13 |
| $HTA_{14}$ | Ca | 37,1 | 6,27 | –25 |
| $HTA_{18}$ | Ca | 36,7 | 6,00 | – 3 |
| base oil | – | 30,82 | 5,38 | –11 |

**Claims**

1. Derivatives of a mixture of monocarboxylic acids, at least part of which are branched-chain acids, selected from the group consisting of
a) esters of phenols or aromatic, aliphatic or cycloaliphatic alcohols having at least 1 to 10 primary or secondary hydroxyl groups;
b) amides of ammonia or of aliphatic, cycloaliphatic or aromatic amines having at least 1 to 15 primary or secondary amino groups;
c) salts of alkali metals or alkaline earth metals, amphoteric metals, heavy metals or ammonium or of a tertiary amino group containing compound, characterized in that the monocarboxylic acids are derived from the reaction product of an $\alpha$-olefin having 6 to 45 carbon atoms and acetic anhydride, and at least 10% by weight of the branched-chain acids conforms to the formula:

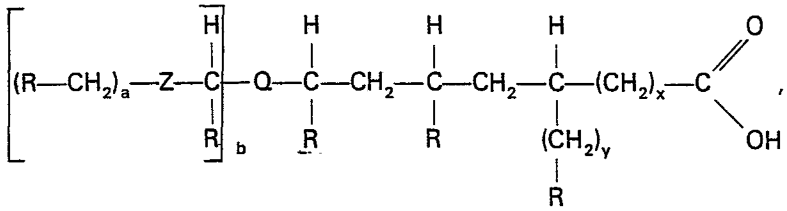

, wherein

y=0 or 2 and
x=0, if y=2
or x=2, if y=0;
$R=CH_3(CH_2)_n$, where n represents an integer of from 3 to 42;
b=0 or 1, where,
if b=0, Q represents a hydrogen atom, and
if b=1, Q represents a $CH_2$-group, and
a=0 or 1, where
if a=0, Z represents a hydrogen atom, and
if a=1, Z represents a $CH_2$-group.

2. Derivatives of a mixture of monocarboxylic acids according to claim 1, characterized in that n represents an integer of from 3 to 17.

3. Derivatives of a mixture of monocarboxylic acids according to claim 1, characterized in that n represents an integer of from 17 to 42.

4. Esters of aliphatic or cycloaliphatic alcohols and a mixture of monocarboxylic acids according to claim 1, characterized in that n represents an integer of from 3 to 11 and the alcohols contain not more than 18 carbon atoms.

5. Esters of a mixture of monocarboxylic acids according to claim 1, characterized in that the aliphatic alcohol is a branched-chain aliphatic polyalcohol having 2 to 4 primary hydroxyl groups.

6. Esters according to claim 5, characterized in that the aliphatic alcohol is neopentyl glycol.

7. Esters according to claim 5, characterized in that the aliphatic alcohol is trimethylol propane.

8. Esters according to claim 5, characterized in that the aliphatic alcohol is pentaerythritol.

9. Esters of a mixture of monocarboxylic acids according to claim 1, characterized in that the aliphatic alcohol is a polyol or ether polyol having 2 to 12 carbon atoms and 2 to 8 primary or secondary hydroxyl groups.

10. Esters according to claim 9, characterized in that they are derived from ethylene glycol, neopentyl glycol, pentaerythritol, dipentaerythritol, tripentaerythritol, glycerol and/or di-, tri-, and/or tetraglycerol.

11. Esters derived from a polyol having at least 2 to 10 primary or secondary hydroxyl groups and a mixture of monocarboxylic acids according to claim 1, characterized in that the esters are prepared from an acid mixture comprising one or more acids of the formula:

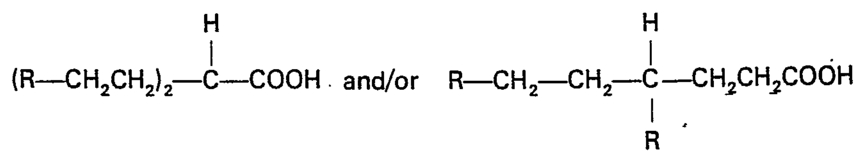

where R has the meaning given in claim 1.

12. Esters derived from a polyol having at least 2 to 10 primary or secondary hydroxyl groups and a mixture of monocarboxylic acids according to claim 1, characterized in that the esters are prepared from an acid mixture comprising unbranched aliphatic monocarboxylic acids having 7 to 47 carbon atoms.

13. Amide derivatives of a polyamine and a mixture of monocarboxylic acids according to claim 1, characterized in that the polyamine corresponds to the formula:

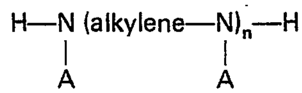

where n represents an integer of from 1 to 10, A is a hydrogen atom or a hydrocarbon group substituted or not with an amino group, and the alkylene group is a lower alkylene group having not more than 8 carbon atoms.

14. Amide derivatives according to claim 13, characterized in that the polyamine is an oligomer of ethylene diamine.

15. Amide derivatives according to claim 14, characterized in that they are obtained from diethylene triamine, triethylene tetramine, tetraethylene pentamine and/or pentaethylene hexamine.

16. Amide derivatives according to claim 1, characterized in that they are obtained from aminoethyl piperazine.

17. A salt of a mixture of monocarboxylic acids according to claim 1, characterized in that the metal is selected from the group of calcium, barium and magnesium.

18. A lubricant composition containing a salt mixture according to claim 1, with the proviso that n represents an integer of from 3 to 26.

19. A lubricant composition for engine oils or polymers containing an overbased formulation comprising a salt mixture according to claim 17.

20. A lubricant composition of which the base fluid is entirely or partly composed of an ester mixture according to one or more of the claims 4—8.

21. A lubricant composition containing a mixture of amide derivatives according to claim 2 and one or more of the claims 13—16.

22. A lubricant composition for polymers, which composition is entirely or partly composed of derivatives according to claim 1.

23. A wax composition which is entirely or partly to replace carnauba wax and/or montan wax and in which derivatives according to claim 3 are included.

**Revendications**

1. Dérivés d'un mélange d'acides monocarboxyliques, dont au moins une partie sont des acides à chaîne ramifiée, choisis dans le groupe constitué par

a) des esters de phénols ou d'alcools aromatiques, aliphatiques ou cyclo-aliphatiques ayant au moins 1 à 10 groupes hydroxyle primaires ou secondaires;

b) des amides d'ammoniac ou d'amines aliphatiques, cyclo-aliphatiques ou aromatiques ayant au moins 1 à 15 groupes amino primaires ou secondaires;

c) des sels de métaux alcalins ou de métaux alcalino-terreux, de métaux amphotères, de métaux lourds ou d'ammonium ou d'un composé contenant un groupe amino tertiaire;

caractérisés en ce que les acids monocarboxyliques sont dérivés du produit de réaction d'une alpha-oléfine ayant 6 à 45 atomes de carbone et d'anhydride acétique, et qu'au moins 10% en poids des acides à chaîne ramifiée sont conformes à la formule:

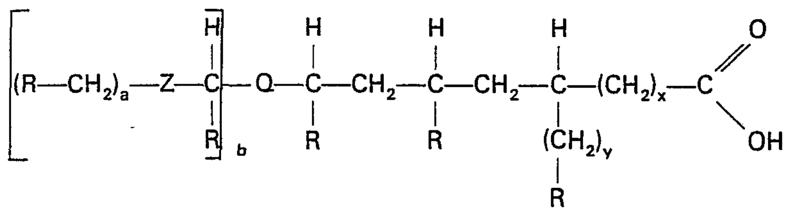

dans laquelle

y=0 ou 2 ou

x=0, si y=2

ou x=2, si y=0;

$R=CH_3(CH_2)_n$, où n représente un nombre entier de 3 à 42;

b=0 ou 1, où

si b=0, Q représente un atome d'hydrogène, et

si b=1, Q représente un groupe $CH_2$, et

a=0 ou 1, où

si a=0, Z représente un atome d'hydrogène, et

si a=1, Z représente un groupe $CH_2$.

2. Dérivés d'un mélange d'acides monocarboxyliques selon la revendication 1, caractérisés en ce que n représente un nombre entier de 3 à 17.

3. Dérivés d'un mélange d'acides monocarboxyliques selon la revendication 1, caractérisés en ce que n représente un nombre entier de 17 à 42.

4. Esters d'alcools aliphatiques ou cyclo-aliphatiques et d'un mélange d'acides moncarboxyliques selon la revendication 1, caractérisés en ce que n représente un nombre entier de 3 à 11 et que les alcools ne contiennent pas plus de 18 atomes de carbone.

5. Esters d'un mélange d'acides monocarboxyliques selon la revendication 1, caractérisé en ce que l'alcool aliphatique est un polyalcool aliphatique à chaîne ramifiée ayant 2 à 4 groupes hydroxyle primaires.

6. Esters selon la revendication 5, caractérisés en ce que l'alcool aliphatique est le néopentylglycol.

7. Esters selon la revendication 5, caractérisés en ce que l'alcool aliphatique est le triméthylolpropane.

8. Esters selon la revendication 5, caractérisés en ce que l'alcool aliphatique est le penta-érythritol.

9. Esters d'un mélange d'acides monocarboxyliques selon la revendication 1, caractérisés en ce que l'alcool aliphatique est un polyol ou éther polyol ayant 2 à 12 atomes de carbone et 2 à 8 groupes hydroxyle primaires ou secondaires.

10. Esters selon la revendication 9, caractérisés en ce qu'ils sont dérivés d'éthylèneglycol, de néopentylglycol, de penta-érythritol, de dipenta-érythritol, de tripentaérythritol, de glycérol et/ou de di-, tri- et/ou tétraglycérol.

11. Esters dérivés d'un polyol ayant au moins 2 à 10 groupes hydroxyle primaires ou secondaires et d'un mélange d'acides monocarboxyliques selon la revendication 1, caractérisés en ce que les esters sont préparés à partir d'un mélange d'acides comprenant un ou plusieurs acides de la formule:

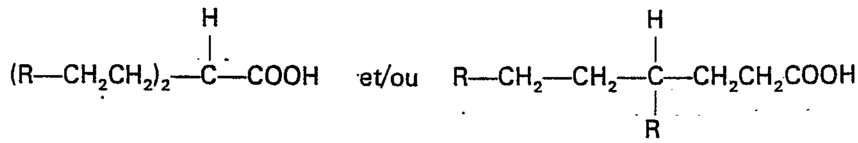

où R a la signification indiquée dans la revendication 1.

12. Esters dérivés d'un polyol ayant au moins 2 à 10 groupes hydroxyle primaires ou secondaires et d'un mélange d'acides monocarboxyliques selon la revendication 1, caractérisés en ce que les esters sont préparés à partir d'un mélange d'acides comprenant des acides monocarboxyliques aliphatiques non ramifiés ayant 7 à 47 atomes de carbone.

13. Dérivés amide d'une polyamine et d'un mélange d'acides monocarboxyliques selon la revendication 1, caractérisé en ce que la polyamine correspond à la formule:

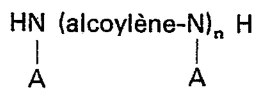

dans laquelle n représente un nombre entier de 1 à 10, A est un atome d'hydrogène ou un groupe d'hydrocarbure substitué ou non par un groupe amino et le groupe alcoylène est un groupe alcoylène inférieur n'ayant pas plus de 8 atomes de carbone.

14. Dérivés amide selon la revendication 13, caractérisés en ce que la polyamine est un oligomère d'éthylènediamine.

15. Dérivés amide selon la revendication 14, caractérisés en ce qu'ils sont obtenus à partir de diéthylènetriamine, de triéthylènetétramine, de tétraéthylènepentamine et/ou de pentaéthylène-hexamine.

16. Dérivés amide selon la revendication 1, caractérisés en ce qu'ils sont obtenus à partir d'aminoéthylpipérazine.

17. Un sel d'un mélange d'acides monocarboxyliques selon la revendication 1, caractérisé en ce que le métal est choisi parmi le calcium, le baryum et le magnésium.

18. Une composition lubrifiante contenant un mélange de sels selon la revendication 1, avec la condition que n représente un nombre entier de 3 à 26.

19. Une composition lubrifiante pour huiles moteur ou pour polymères contenant une composition superbasique comprenant un mélange de sels selon la revendication 17.

20. Une composition lubrifiante dont le fluide de base est composé entièrement ou partiellement d'un mélange d'esters selon une ou plusieurs des revendications 4 à 8.

21. Une composition lubrifiante comprenant un mélange de dérivés amide selon la revendication 2 et une ou plusieurs des revendications 13 à 16.

22. Une composition lubrifiante pour polymères, qui est composée entièrement ou partiellement de dérivés selon la revendication 1.

23. Une composition de cire prévue pour remplacer entièrement ou partiellement la cire de Carnauba et/ou la cire de lignite et dans laquelle des dérivés selon la revendication 3 sont inclus.

**Patentansprüche**

1. Derivate eines Gemisches von Monocarbonsäuren, von denen zumindest ein Teil verzweigte Säuren sind, ausgewählt aus der Gruppe bestehend aus

a) Estern von Phenolen oder aromatischen, aliphatischen oder cycloaliphatischen Alkoholen mit mindestens 1 bis 10 primären oder sekundären Hydroxylgruppen;

b) Amiden von Ammoniak oder aliphatischen, cycloaliphatischen oder aromatischen Aminen mit mindestens 1 bis 15 primären oder sekundären Aminogruppen;

c) Salzen von Alkalimetallen oder Erdalkalimetallen, amphoteren Metallen, Schwermetallen oder Ammonium oder von einer eine tertiäre Aminogruppen enthaltenden Verbindung, dadurch gekennzeichnet, daß sich die Monocarbonsäuren von dem Reaktionsprodukt eines $\alpha$-Olefins mit 6 bis 45

Kohlenstoffatomen und Essigsäureanhydrid ableiten und mindestens 10 Gewichtsprozent der verzweigten Säuren der Formel entsprechen:

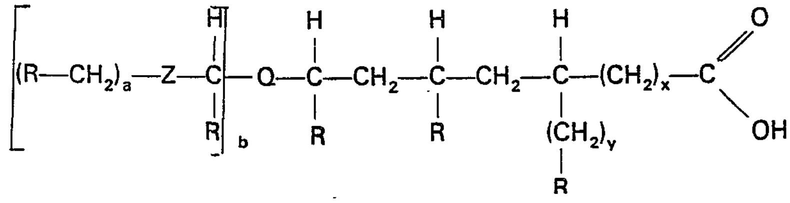

in der
$y=0$ oder 2 und
$x=0$, wenn $y=2$ oder
$x=2$, wenn $y=0$,
$R=CH_3(CH_2)_n$, wobei n eine ganze Zahl von 3 bis 42 bedeutet,
$b=0$ oder 1, wobei
wenn $b=0$, Q ein Wasserstoffatom bedeutet und
wenn $b=1$, Q eine $CH_2$-Gruppe bedeutet, und
$a=0$ oder 1, wobei
wenn $a=0$, Z ein Wasserstoffatom bedeutet oder
wenn $a-1$, Z eine $CH_2$-Gruppe bedeutet.

2. Derivate eines Gemisches von Monocarbonsäuren gemäß Anspruch 1, dadurch gekennzeichnet, daß n eine ganze Zahl von 3 bis 17 bedeutet.

3. Derivate eines Gemisches von Monocarbonsäuren gemäß Anspruch 1, dadurch gekennzeichnet, daß n eine ganze Zahl von 17 bis 42 bedeutet.

4. Ester von aliphatischen oder cycloaliphatischen Alkoholen und eines Gemisches von Monocarbonsäuren gemäß Anspruch 1, dadurch gekennzeichnet, daß n eine ganze Zahl von 3 bis 11 bedeutet und die Alkohole nicht mehr als 18 Kohlenstoffatome enthalten.

5. Ester eines Gemisches von Monocarbonsäuren gemäß Anspruch 1, dadurch gekennzeichnet, daß der aliphatische Alkohol ein verzweigter aliphatischer Polyalkohol mit 2 bis 4 primären Hydroxylgruppen ist.

6. Ester gemäß Anspruch 5, dadurch gekennzeichnet, daß der aliphatische Alkohol Neopentylglykol ist.

7. Ester gemäß Anspruch 5, dadurch gekennzeichnet, daß der aliphatische Alkohol Trimethylolpropan ist.

8. Ester gemäß Anspruch 5, dadurch gekennzeichnet, daß der aliphatische Alkohol Pentaerythrit ist.

9. Ester eines Gemisches von Monocarbonsäuren gemäß Anspruch 1, dadurch gekennzeichnet, daß der aliphatische Alkohol ein Polyol oder Äther-polyol mit 2 bis 12 Kohlenstoffatomen und 2 bis 8 primären oder sekundären Hydroxylgruppen ist.

10. Ester gemäß Anspruch 9, dadurch gekennzeichnet, daß sie sich von Äthylenglykol, Neopentylglykol, Pentaerythrit, Dipentaerythrit, Tripentaerythrit, Glycerin und/oder Di-, Tri- und/oder Tetraglycerin ableiten.

11. Ester, die sich von einem Polyol mit mindestens 2 bis 10 primären oder sekundären Hydroxylgruppen und einem Gemisch von Monocarbonsäuren gemäß Anspruch 1 ableiten, dadurch gekennzeichnet, daß die Ester aus einem Säuregemisch hergestellt werden, das eine oder mehrere Säuren der Formel:

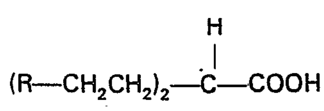 und/oder 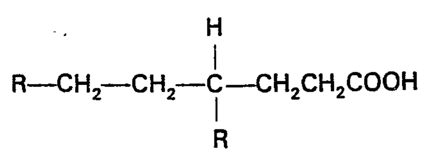

enthält, in der R die in Anspruch 1 angegebene Bedeutung hat.

12. Ester, die sich von einem Polyol mit mindestens 2 bis 10 primären oder sekundären Hydroxylgruppen und einem Gemisch von Monocarbonsäuren gemäß Anspruch 1 ableiten, dadurch gekennzeichnet, daß die Ester aus einem Säuregemisch, das unverzweigte aliphatische Monocarbonsäuren mit 7 bis 47 Kohlenstoffatomen enthält, hergestellt werden.

13. Amidderivate eines Polyamins und eines Gemisches von Monocarbonsäuren gemäß Anspruch 1, dadurch gekennzeichnet, daß das Polyamin der Formel entspricht:

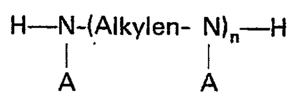

in der n eine ganze Zahl von 1 bis 10 bedeutet, A ein Wasserstoffatom oder eine Kohlenwasserstoffgruppe ist, die unsubstituiert oder mit einer Aminogruppe substituiert ist, und die Alkylengruppe eine niedere Alkylengruppe ist, die nicht mehr als 8 Kohlenstoffatome hat.

14. Amidderivate gemäß Anspruch 13, dadurch gekennzeichnet, daß das Polyamin ein Oligomeres von Äthylendiamin ist.

15. Amidderivate gemäß Anspruch 14, dadurch gekennzeichnet, daß sie aus Diäthylentriamin, Triäthylentetramin, Tetraäthylenpentamin und/oder Pentaäthylenhexamin erhalten werden.

16. Amidderivate gemäß Anspruch 1, dadurch gekennzeichnet, daß sie aus Aminoäthylpiperazin erhalten werden.

17. Ein Salz eines Gemisches von Monocarbonsäuren gemäß Anspruch 1, dadurch gekennzeichnet, daß das Metall aus der Gruppe von Calcium, Barium und Magnesium ausgewählt wird.

18. Eine Schmiermittel-Zusammensetzung, enthaltend ein Salzgemisch gemäß Anspruch 1, mit der Maßgabe, daß n eine ganze Zahl von 3 bis 26 bedeutet.

19. Eine Schmiermittel-Zusammensetzung für Maschinenöle oder Polymere, eine überbasisch eingestellte Formulierung enthaltend, die ein Salzgemisch gemäß Anspruch 17 enthält.

20. Eine Schmiermittel-Zusammensetzung, in der die Grundflüssigkeit ganz oder teilweise aus einem Estergemisch gemäß einem oder mehreren der Ansprüche 4 bis 8 besteht.

21. Eine Schmiermittel-Zusammensetzung, enthaltend ein Gemisch von Amidderivaten gemäß Anspruch 2 und einem oder mehreren der Ansprüche 13 bis 16.

22. Eine Schmiermittel-Zusammensetzung für Polymere, die ganz oder teilweise aus Derivaten gemäß Anspruch 1 besteht.

23. Eine Wachs-Zusammensetzung, die für den vollständigen oder teilweisen Ersatz von Carnaubawachs und/oder Montanwachs bestimmt ist, und in die Derivate gemäß Anspruch 3 einverleibt sind.